# EUROPEAN PATENT APPLICATION

(11) **EP 2 327 801 A1**
(43) Date of publication of application: **01.06.2011**
(21) Application number: 10197403.8
(22) Date of filing: 24.04.2008
(51) Int. Cl.: C12Q 1/68

(54) **Functional toll-like receptors (TLR) on melanocytes and melanoma cells and uses thereof**

(30) Priority: 24.04.2007 US 913742 P
(62) Divisional of application: 08746816.1
(71) Applicant: John Wayne Cancer Institute, Santa Monica, CA 90404 (US)
(72) Inventor: Hoon, Dave S.B., Los Angeles, CA 90025 (US); Goto, Yasufumi, Santa Monica, CA 90404 (US)
(74) Representative: Viering, Jentschura & Partner

(57) **Abstract**

The invention related to a method of detecting Toll-like receptors (TLR) gene expression or protein activity in a melanocyte or melanoma cell. Also disclosed are a method of modulating TLR gene expression or protein activity in a melanocyte or melanoma cell by contacting the cell with a TLR modulating agent and a method of inhibiting melanoma cell migration (e.g., spreading) by contacting the cell with a TLR inhibitor.

SEQUENCE LISTING

<110>  John Wayne Cancer Institute

<120>  FUNCTIONAL TOLL-LIKE RECEPTORS (TLR) ON MELANOCYTES AND MELANOMA
       CELLS AND USES THEREOF

<130>  P44320

<140>
<141>  2008-04-24

<150>  US 60/913,742
<151>  2007-04-24

<160>  39

<170>  PatentIn version 3.4

<210>  1
<211>  20
<212>  DNA
<213>  Artificial

<220>
<223>  Primer

<400>  1
gggtgtgaac catgagaagt                                                  20


<210>  2
<211>  19
<212>  DNA
<213>  Artificial

<220>
<223>  Primer

<400>  2
gactgtggtc atgagtcct                                                   19


<210>  3
<211>  21
<212>  DNA
<213>  Artificial

<220>
<223>  Primer

<400>  3
agttgtcagc gatgtgttcg g                                                21


<210>  4
<211>  22
<212>  DNA
<213>  Artificial

<220>
<223>  Primer

<400>  4
gatcaagtac cttgatcctg gg                                               22

<210>  5
<211>  21
<212>  DNA
<213>  Artificial

<220>
<223>  Primer

<400>  5
tcaactggta gttgtgggtt g                                                21


<210>  6
<211>  21
<212>  DNA
<213>  Artificial

<220>
<223>  Primer

<400>  6
caagacagag aagcctgatt g                                                21


<210>  7
<211>  22
<212>  DNA
<213>  Artificial

<220>
<223>  Primer

<400>  7
gacccattat gcaaaagatt ca                                               22


<210>  8
<211>  20
<212>  DNA
<213>  Artificial

<220>
<223>  Primer

<400>  8
gcaaacagag tgcatggttc                                                  20


<210>  9
<211>  18
<212>  DNA
<213>  Artificial

<220>
<223>  Primer

<400>  9
ggatttatcc aggtgtga                                                    18


<210>  10
<211>  17
<212>  DNA
<213>  Artificial

<220>
<223>  Primer

<400>  10
tcccagggct aaactct                                                    17


<210>  11
<211>  21
<212>  DNA
<213>  Artificial

<220>
<223>  Primer

<400>  11
acaacttagc acggctctgg a                                               21


<210>  12
<211>  22
<212>  DNA
<213>  Artificial

<220>
<223>  Primer

<400>  12
gaaaccccag agaacgagtc ag                                              22


<210>  13
<211>  20
<212>  DNA
<213>  Artificial

<220>
<223>  Primer

<400>  13
atcctcatgc cttcaggaaa                                                 20


<210>  14
<211>  20
<212>  DNA
<213>  Artificial

<220>
<223>  Primer

<400>  14
accagaagag actgggctgt                                                 20


<210>  15
<211>  22
<212>  DNA
<213>  Artificial

<220>
<223>  Primer

<400>  15
gataacaatg tcacagccgt cc                                              22


<210>  16
<211>  23
<212>  DNA

<213>  Artificial

<220>
<223>  Primer

<400>  16
gttcctggag tttgttgatg ttc                                              23


<210>  17
<211>  24
<212>  DNA
<213>  Artificial

<220>
<223>  Primer

<400>  17
gtgtcaccca aactgccaag ctcc                                             24


<210>  18
<211>  23
<212>  DNA
<213>  Artificial

<220>
<223>  Primer

<400>  18
gatccagcac cttcagatga ggc                                              23


<210>  19
<211>  24
<212>  DNA
<213>  Artificial

<220>
<223>  Primer

<400>  19
taccaacatc ctgatgctag actc                                             24


<210>  20
<211>  24
<212>  DNA
<213>  Artificial

<220>
<223>  Primer

<400>  20
taggacaaca gcagatactc cagg                                             24


<210>  21
<211>  21
<212>  DNA
<213>  Artificial

<220>
<223>  Primer

<400>  21
tatgacagca gagggtgatg c                                                21

<210> 22
<211> 21
<212> DNA
<213> Artificial

<220>
<223> Primer

<400> 22
tgcgggaacc tttcttagag a 21


<210> 23
<211> 19
<212> DNA
<213> Artificial

<220>
<223> Primer

<400> 23
aaaactgtga acctgtggt 19


<210> 24
<211> 21
<212> DNA
<213> Artificial

<220>
<223> Primer

<400> 24
ttcaagcaaa agtgtgagag a 21


<210> 25
<211> 21
<212> DNA
<213> Artificial

<220>
<223> Primer

<400> 25
attcactgca gggatggatc t 21


<210> 26
<211> 20
<212> DNA
<213> Artificial

<220>
<223> Primer

<400> 26
attgctcgaa tgtggaaacc 20


<210> 27
<211> 20
<212> DNA
<213> Artificial

<220>
<223> Primer

<400>  27
tcgagagctt cagacaagca                                                    20

<210>  28
<211>  20
<212>  DNA
<213>  Artificial

<220>
<223>  Primer

<400>  28
ttcctcctca actggtttcc                                                    20

<210>  29
<211>  17
<212>  DNA
<213>  Artificial

<220>
<223>  Primer

<400>  29
acttaccgcc gccttct                                                       17

<210>  30
<211>  17
<212>  DNA
<213>  Artificial

<220>
<223>  Primer

<400>  30
cgaccttgcc atccact                                                       17

<210>  31
<211>  20
<212>  DNA
<213>  Artificial

<220>
<223>  Primer

<400>  31
gctggggatc ctagatttca                                                    20

<210>  32
<211>  18
<212>  DNA
<213>  Artificial

<220>
<223>  Primer

<400>  32
ccagcctttc tcctgtgg                                                      18

<210>  33
<211>  19
<212>  DNA

<213>  Artificial

<220>
<223>  Primer

<400>  33
cctctgtagg ccgactgct                                                    19


<210>  34
<211>  19
<212>  DNA
<213>  Artificial

<220>
<223>  Primer

<400>  34
ttggcaatcc tcctcaatg                                                    19


<210>  35
<211>  24
<212>  DNA
<213>  Artificial

<220>
<223>  Probe

<400>  35
cagcaatgcc tcctgcacca ccaa                                              24


<210>  36
<211>  25
<212>  DNA
<213>  Artificial

<220>
<223>  Probe

<400>  36
ttcttccttg gagaggctga tgatg                                             25


<210>  37
<211>  25
<212>  DNA
<213>  Artificial

<220>
<223>  Probe

<400>  37
ctggaatctc ctcaaggaaa accaa                                             25


<210>  38
<211>  25
<212>  DNA
<213>  Artificial

<220>
<223>  Probe

<400>  38
tcctgtcaat attaaggtag agagg                                             25

<210>  39
<211>  20
<212>  DNA
<213>  Artificial

<220>
<223>  Probe

<400>  39
tgcttaccaa gctgggccgc                                                    20

## Description

### RELATED APPLICATION

This application claims priority to U.S. Provisional Application Serial No. 60/913,742, filed on April 24, 2007, the content of which is incorporated herein by reference in its entirety.

### FIELD OF THE INVENTION

The present invention relates in general to Toll-like receptors (TLR). More specifically, the invention relates to detection of TLR gene expression and protein activity in melanocytes and melanoma cells and use of TLR as a target for treatment of melanoma.

### BACKGROUND OF THE INVENTION

Toll-like receptors (TLRs), the mammalian homologues of the *Drosophila* Toll protein, play a critical role in inflammation and innate and adaptive immunity to many human diseases (1, 2). It has become clear that microbial components activate the innate and adaptive immune systems through TLRs by ligation of pathogen-associated molecular patterns (PAMPs) (3, 4). Recently, studies have focused on understanding the relation between TLRs and adaptive immune responses against cancer. However, only a few studies have focused on TLR expression and its functions by cancer cells (5, 6); the role of TLRs in tumor progression is still unclear.

Studies have demonstrated that innate immune cells produce proinflammatory cytokines in the microenvironment of chronic inflammation, whereby TLR activation triggers this response. Inflammation can have a positive effect in eradicating disease; however it can exacerbate disease due to chronic stimulation. A functional link between chronic inflammation and cancer has long been suspected (7, 8). Chronic inflammatory diseases, such as ulcerative colitis and chronic hepatitis, are, respectively, risk factors for the development of colorectal carcinoma and hepatocellular carcinoma. Most cancer tissues show signs of inflammation, such as the presence of innate immune cells, proinflammatory cytokines, and chemokines in the microenvironment of tumors (9). Innate immune responses may contribute to cancer progression by producing inflammatory cytokines and growth factors, causing chronic inflammation (10, 11). Many unanswered questions need to be answered on the role of inflammation and tumor progression. Primary melanomas have been shown to induce inflammation and have ulcerations, the latter being a very poor prognostic factor.

### SUMMARY OF THE INVENTION

The present invention is based, at least in part, upon the unexpected discovery of functional Toll-like receptors (TLRs) on human melanoma and melanocytes.

Accordingly, in one aspect, the invention features a method of detecting TLR gene expression or protein activity in a cell. The method comprises providing a melanocyte or melanoma cell and detecting the expression of a TLR gene or the activity of a TLR protein in the cell. In some embodiments, the melanoma cell is isolated from a melanoma tumor specimen.

The expression of the TLR gene may be detected at the mRNA or protein level, for example, by reverse-transcription polymerase chain reaction (RT-PCR), quantitative real-time reverse-transcription polymerase chain reaction (qRT), or immunostaining.

The activity of the TLR protein may be detected by the binding and activation of the TLR protein by its ligand, for example, the binding and activation of TLR2 by zymosan, the binding and activation of TLR3 by poly-inosinic acid:poly-cytidylic acid (PIC) or mRNA from human tumor cells and lymphocytes, and the binding and activation of TLR4 by lipopolysaccharide (LPS).

The activity of the TLR protein may also be detected by the increased expression of the TLR gene, another TLR gene, a TLR adaptor gene, or a TLR effector gene upon the binding and activation of the TLR protein by its ligand or contacting the cell with a supernatant from phytohemagglutinin-L (PHA-L)-treated peripheral blood lymphocytes (PBL) cells.

The activity of the TLR protein may further be detected by the increased expression of a signaling gene downstream of TLR upon the binding and activation of the TLR protein by its ligand.

Alternatively, the activity of the TLR protein may be detected by the increased migration of the cell upon the binding and activation of the TLR protein by its ligand.

In another aspect, the invention features a method of modulating TLR gene expression or protein activity in a cell. The method comprises providing a melanocyte or melanoma cell and contacting the cell with a TLR modulating agent, thereby increasing or decreasing the expression of a TLR gene or the activity of a TLR protein in the cell. In some embodiments, the TLR modulating agent modulates the interaction between the TLR protein and a TLR ligand, adaptor, or effector, or a signaling gene downstream of TLR.

Also within the invention is a method of inhibiting cell migration. The method comprises providing a melanoma cell and contacting the cell with a TLR inhibitor that decreases the expression of a TLR gene or the activity of a TLR protein in the cell, thereby inhibiting the migration of the cell. In some embodiments, the TLR inhibitor inhibits the interaction between the TLR protein and a TLR ligand, adaptor, or effector, or a signaling gene downstream of TLR.

A TLR gene or protein may be TLR1, 2, 3, 4, 5, 6, 7, 8, 9, or 10. A TLR adaptor gene or protein may be MyD88 or CD14. A TLR effector gene or protein may be NFkB1, NFkB2, IRF1, or IRF3. Examples of a signaling gene downstream of TLR include, but are not limited to, proinflammatory cytokine genes (e.g., IL6, TNFα, IL1, IFNα, IFNβ, and G-CSF), chemokine genes (e.g., CCL2 and CXCL10), anti-inflammatory cytokine genes (e.g., IL10), COX-2, and oncogenes (e.g., JUN).

Unless otherwise defined, all technical and scientific terms used herein have the same meaning as commonly understood by one of ordinary skill in the art to which this invention pertains. In case of conflict, the present document, including definitions, will control. The materials, methods, and examples disclosed herein are illustrative only and not intended to be limiting. Other features, objects, and advantages of the invention will be apparent from the description and the accompanying drawings, and from the claims.

### BRIEF DESCRIPTION OF THE FIGURES

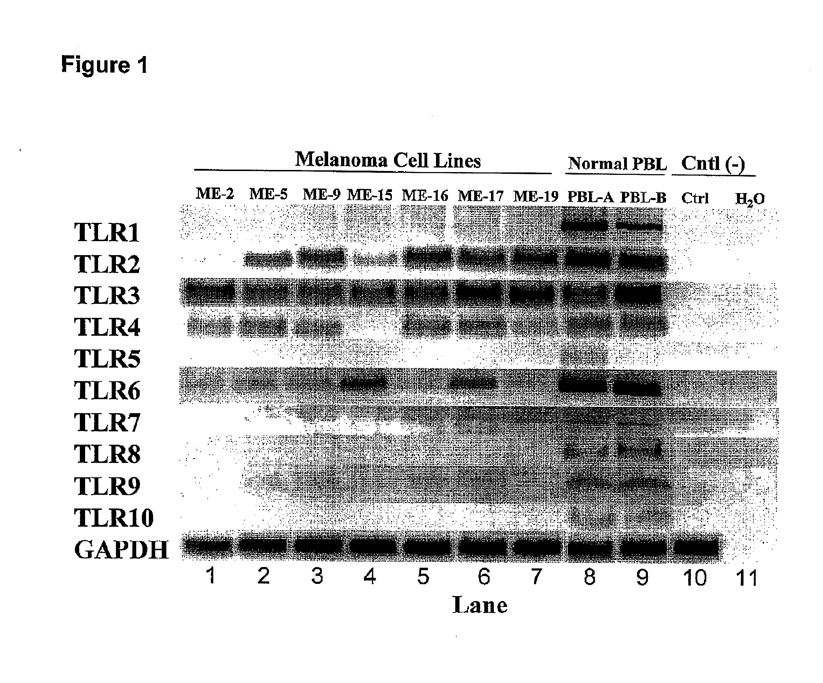

Figure 1. Expression of TLRs (TLR1-10) in 7 human melanoma lines and 2 normal donor PBLs was analyzed by RT-PCR and gel electrophoresis. Colon cancer cell line SW480 was used as a negative control (Cntl).

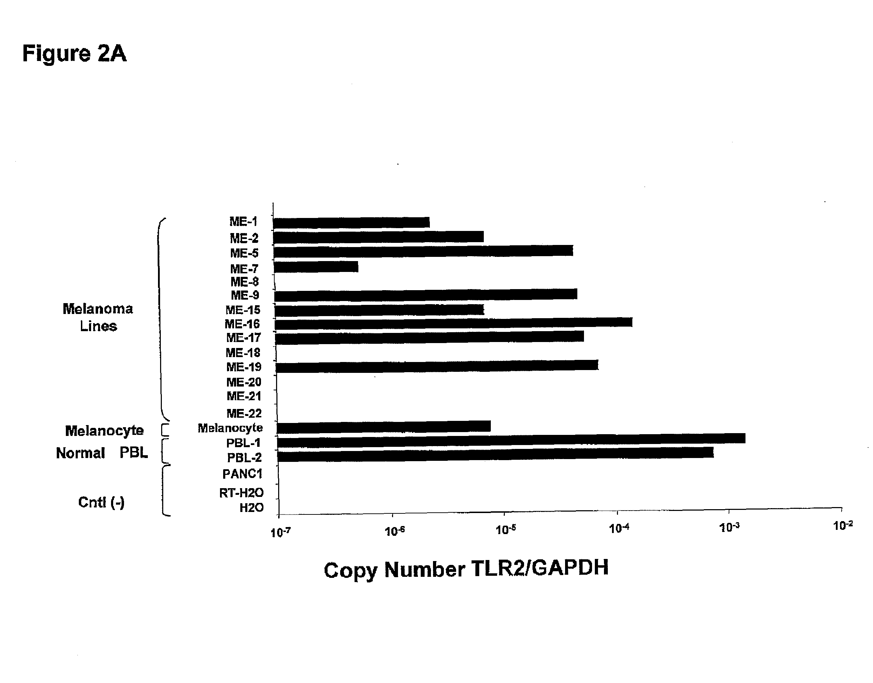

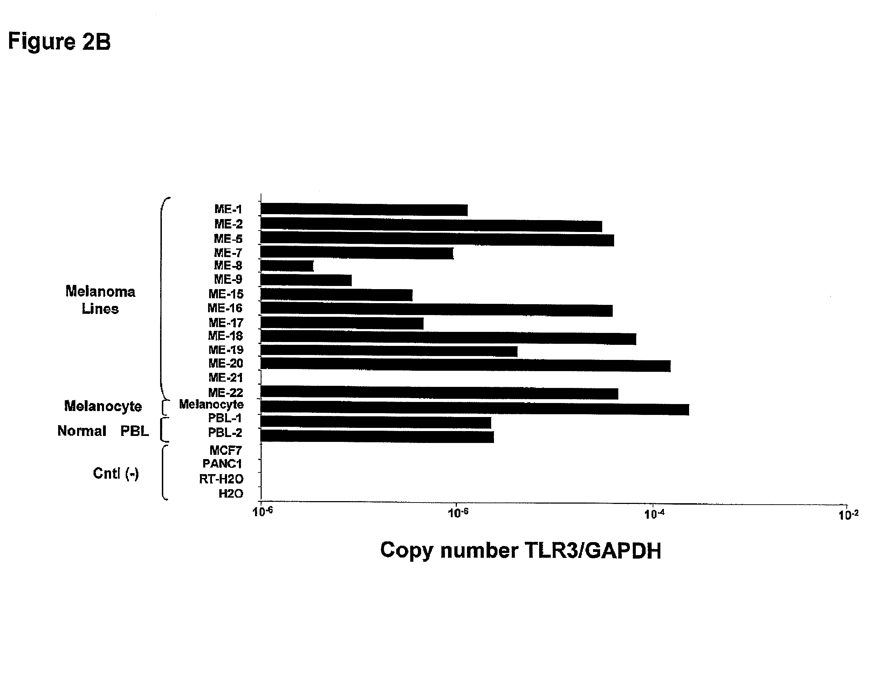

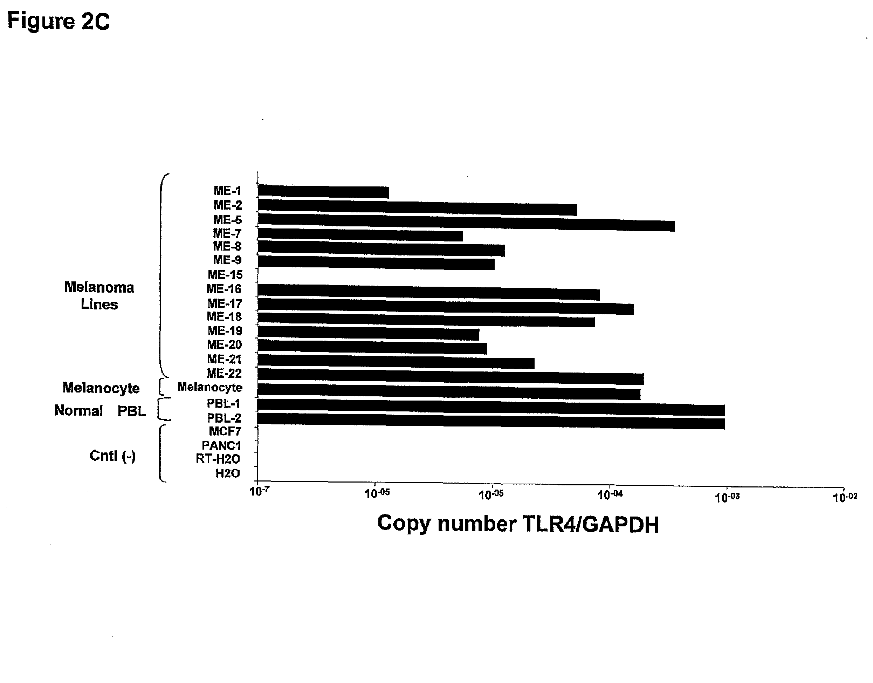

Figure 2. Expression of TLRs in melanoma lines, melanocytes, and healthy donor PBLs was analyzed by qRT. The Y axis is the copy number of each gene over the copy number of the housekeeping gene GAPDH. A: Expression of TLR2; B: Expression of TLR3; and C: Expression of TLR4.

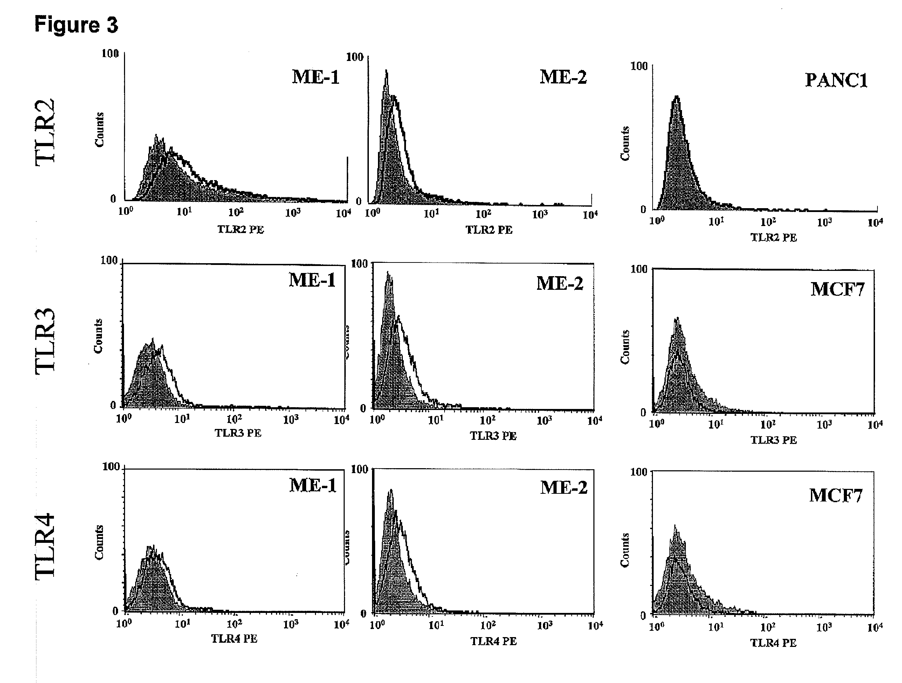

Figure 3. Expression of TLR2, TLR3, and TLR4 in melanoma lines was analyzed by FACS. Cancer cell lines PANC1 and MCF7 were used as negative controls. Left curve shaded area is isotype controls.

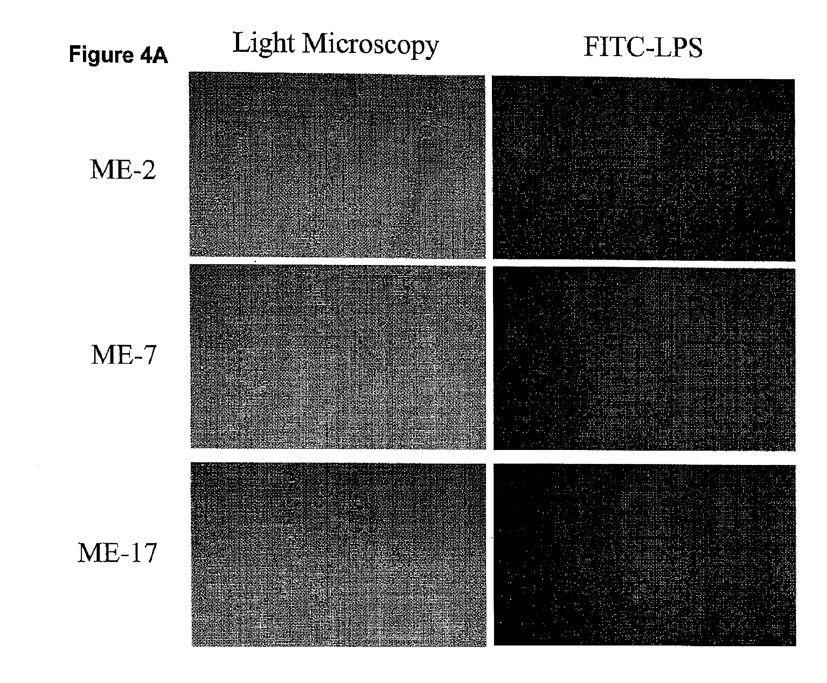

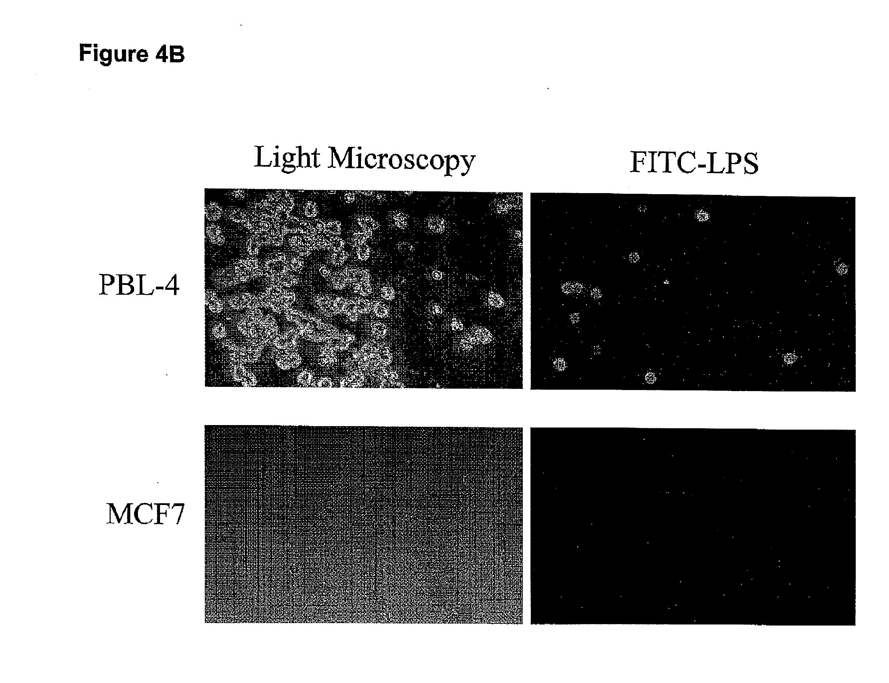

Figure 4. Melanoma cells were stained with FITC-LPS and visualized by fluorescence light microscopy. PBL from healthy individuals was used as a positive control, and MCF7 was used as a negative control.

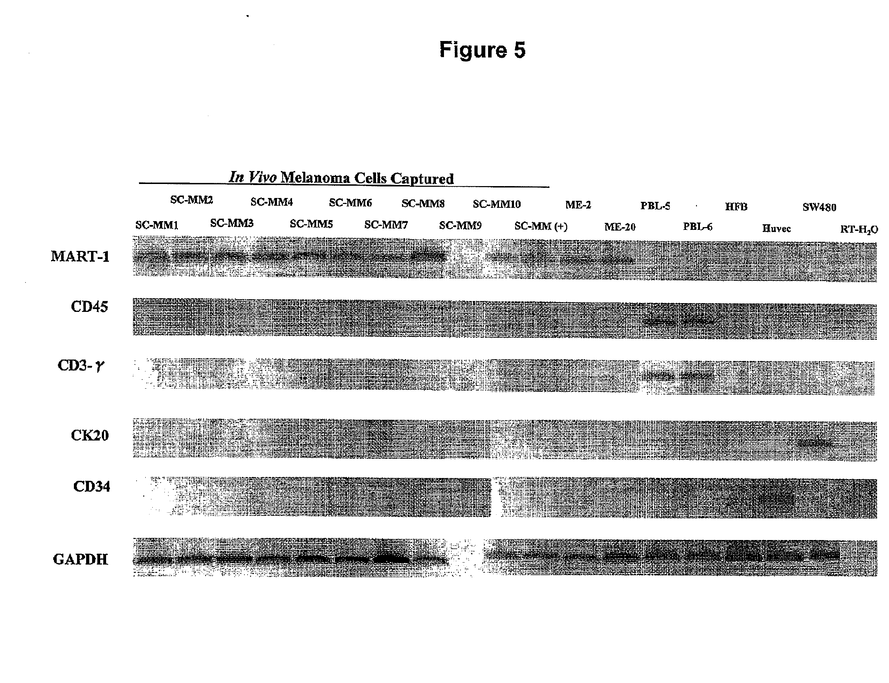

Figure 5. Expression of MART-1 (melanoma marker), CD45 (leukocyte-common antigen), CD3-_{Y} (T cell antigen receptor complex), CK20 (cytokeratin 20), and CD34 (hemapoietic progenitor cell antigen) in 10 primary melanoma cells was analyzed by RT-PCR. SC-MM is a positive control of primary melanoma cells. Melanoma lines (ME2 and ME20), normal donor PBLs (PBL1 and PBL2), HFB, Huvec, and colon cancer line (SW480) were used as positive controls of each marker, respectively.

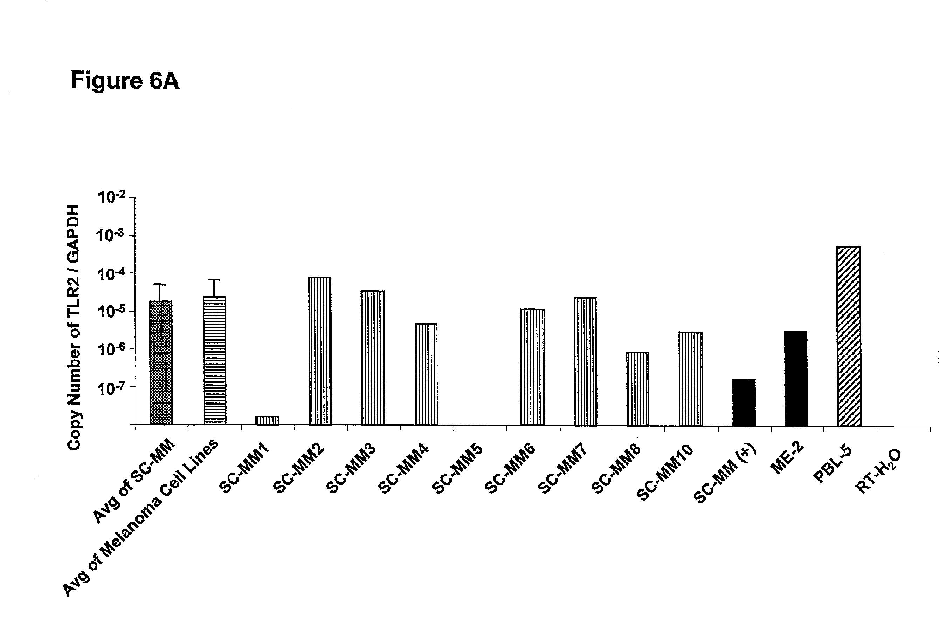

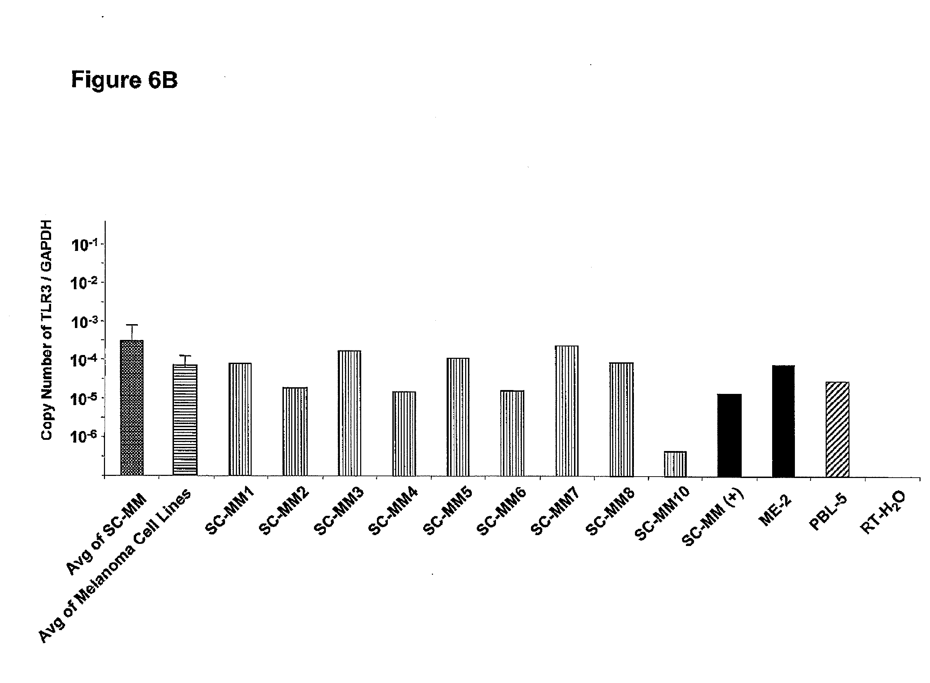

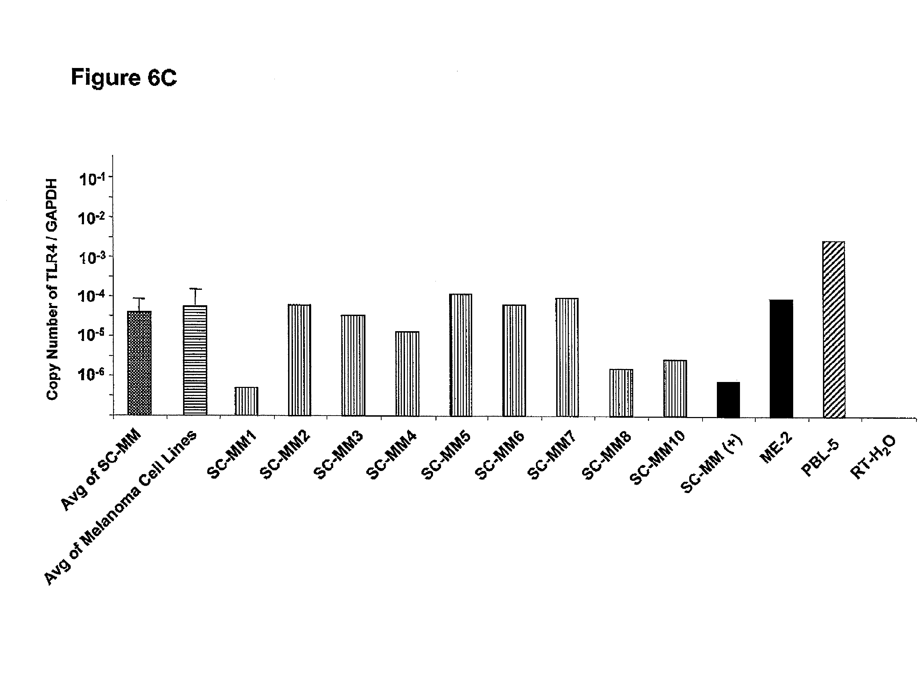

Figure 6. Expression of TLRs in human primary cultured melanoma cells was analyzed by qRT. SC-MM is a positive control of primary melanoma cell, ME2 is a melanoma cell line, and PBL5 is from a healthy individual. An average expression of SC-MM1-SC-MM10 is shown as Avg of SC-MM. An average expression of melanoma cell lines in Figure 2 is shown as Avg of Melanoma Cell Lines. Y axis is the copy number of each gene over the copy number of the housekeeping gene GAPDH. A: Expression of TLR2; B: Expression of TLR3; and C: Expression of TLR4.

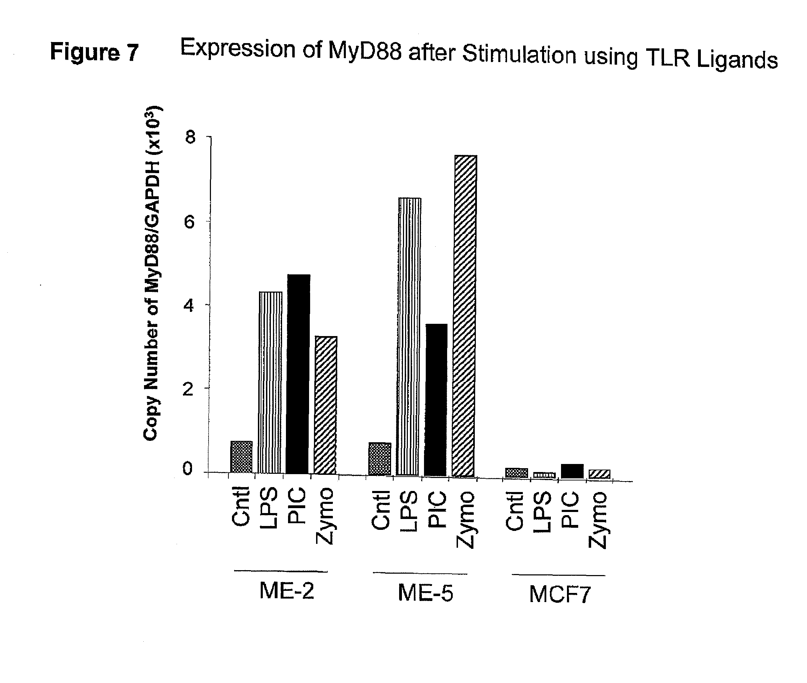

Figure 7. Expression of MyD88 in 2 melanoma lines and one negative control cell line (MCF7) was analyzed by qRT. Each cell line, stimulated by LPS, PIC, and zymosan (Zymo) was compared to each unstimulated cancer line (Cntl). The vertical axis is the copy number of MyD88 over the copy number of the housekeeping gene GAPDH.

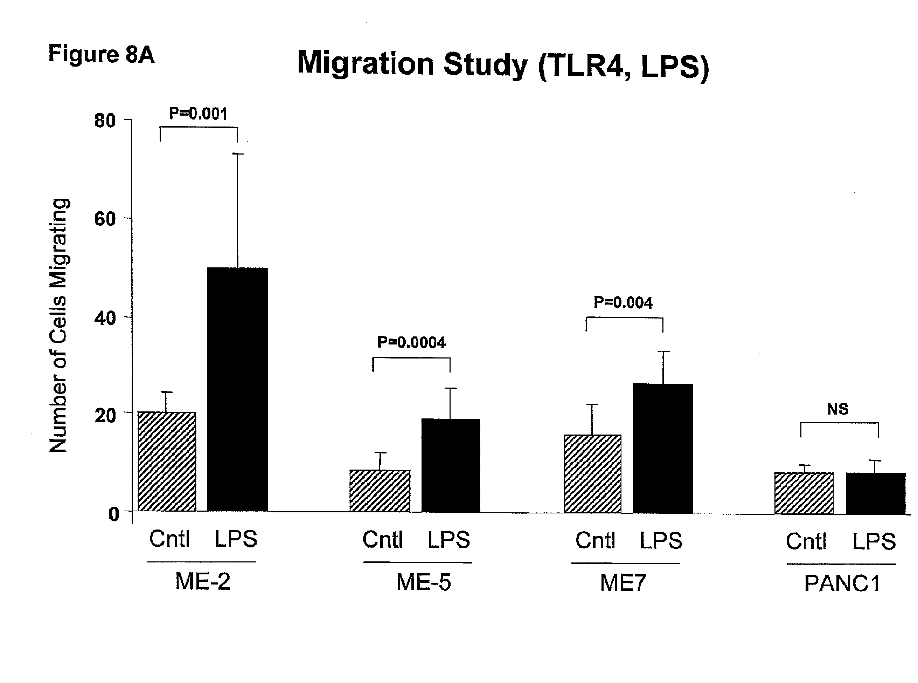

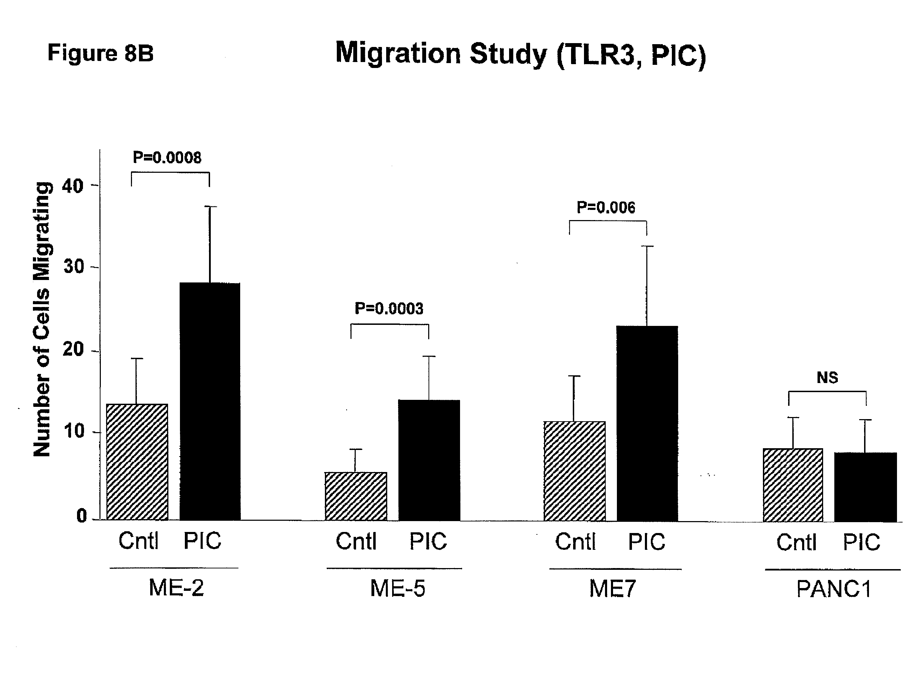

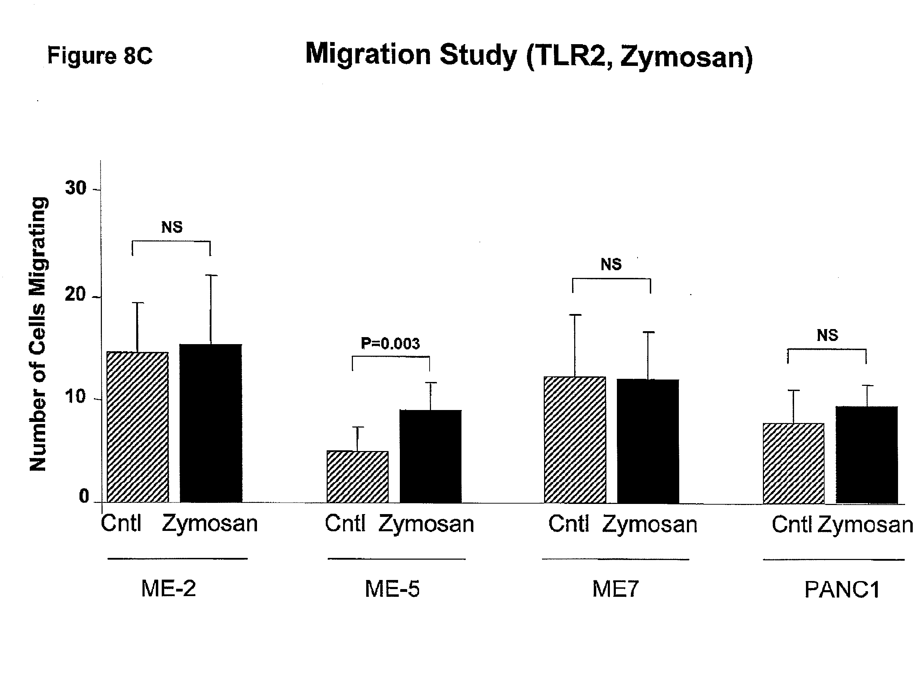

Figure 8. Response to TLR ligands of 2 melanoma cells (ME2 and ME5) and 2 negative control cell lines (breast cancer cell lines, MCF7 and T47D) was assessed by a cell migration assay. The number of migrating cells stimulated by TLR ligands was compared to the number of non-stimulated migrating cells (Ctrl). The number of migrating cells in ten randomly selected fields was counted. One representative experiment out of three is depicted. **A:** LPS as a TLR4 ligand; **B:** PIC as a TLR3 ligand; **C:** Zymosan as a TLR2 ligand.

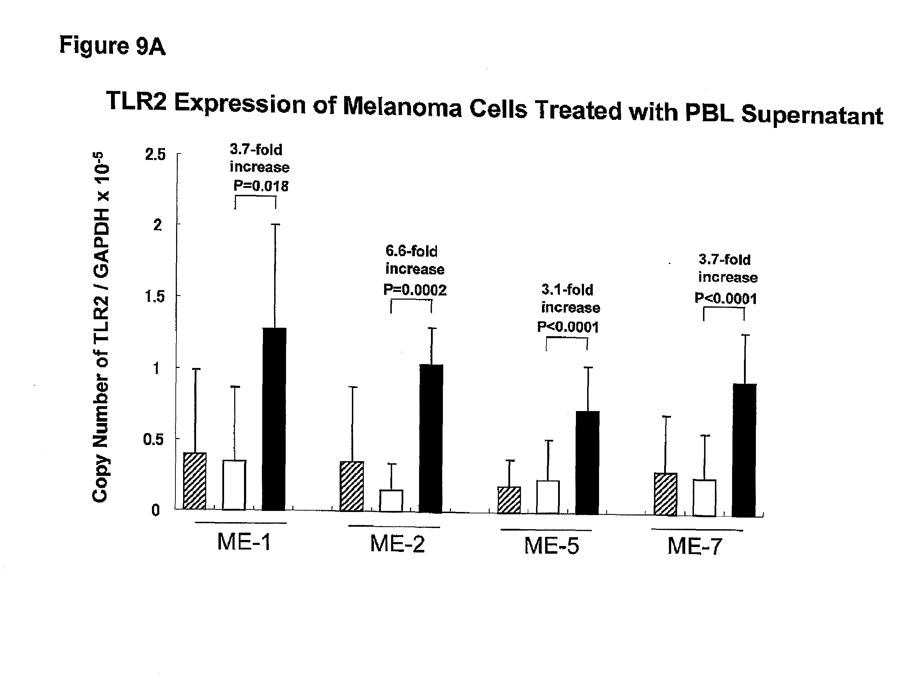

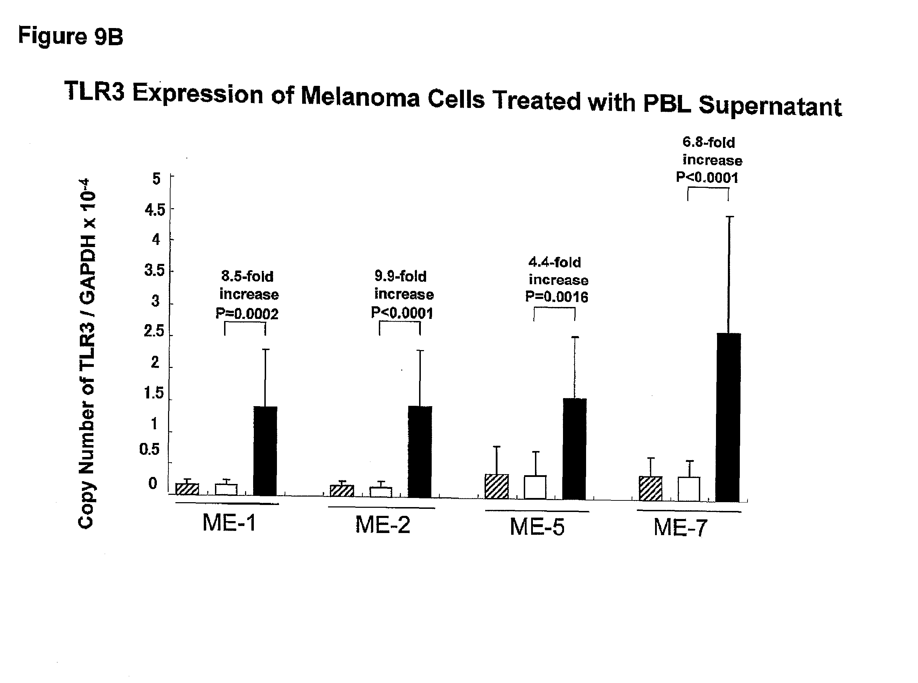

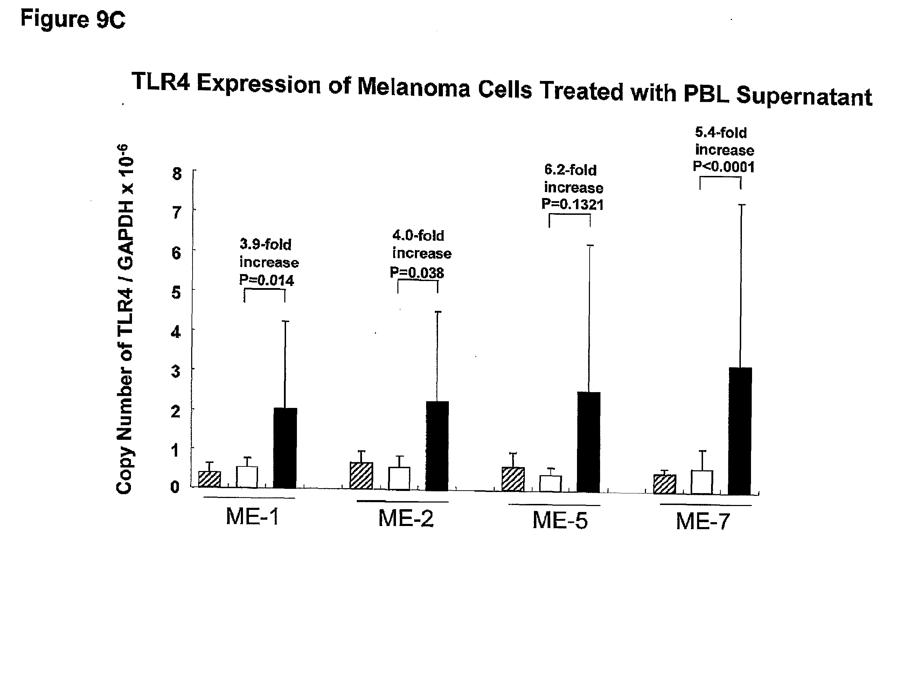

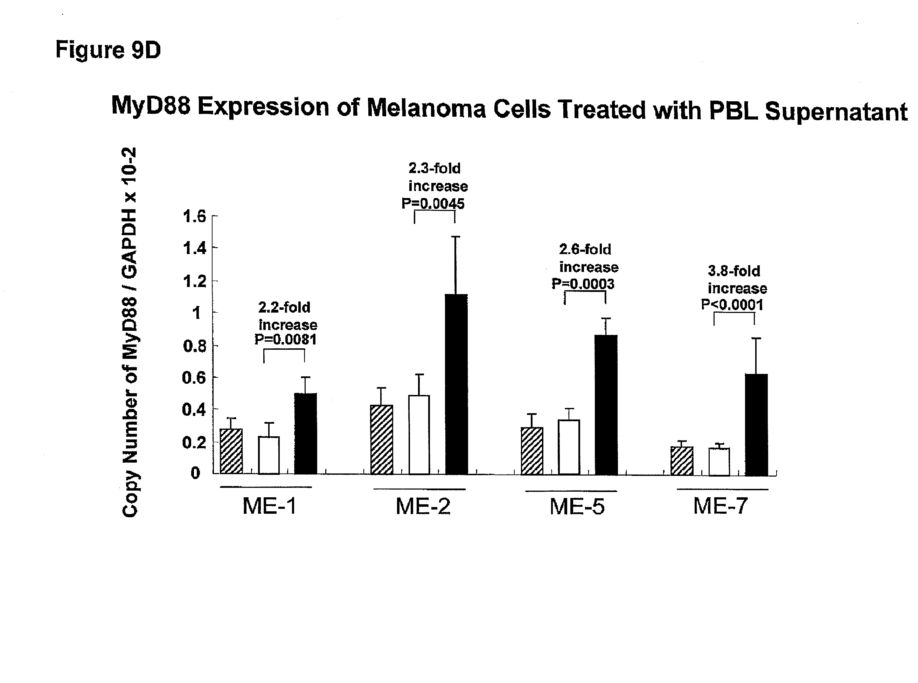

Figure 9. Expression of TLR2, TLR3, TLR4, and MyD88 in 4 melanoma lines (ME1, ME2, ME5, and ME7) cultured with supernatant of PBLs from 4 healthy individuals (PBL-1, PBL-2, PBL3, and PBL4) was analyzed by qRT. ▨ : melanoma cells cultured with a control medium; □: melanoma cells cultured with supernatant from unstimulated PBLs; ■: melanoma cells cultured with supernatant from PHA-L stimulated PBLs. The Y axis is the copy number of each gene over the copy number of the housekeeping gene GAPDH. **A:** Expression of TLR2; **B:** Expression of TLR3; and **C:** Expression of TLR4; **D:** Expression of MyD88; **E:** Expression of TRIF.

### DETAILED DESCRIPTION OF THE INVENTION

Innate immune cells produce proinflammatory cytokines in the environment of chronic inflammation, whereby Toll-like receptors (TLRs) play a significant role. Recently, it has been shown that TLRs have an important role in innate immune responses against cancer. The inventors hypothesized that human melanoma cells express functional TLRs and play a role in inflammation.

To verify that TLRs are expressed and functional on the cell surface in melanoma lines, the inventors performed quantitative real-time reverse-transcription polymerase chain reaction (qRT), fluorescence-activated cell sorting (FACS) analysis, and fluorescence microscopy by FITC-LPS. Melanoma cells *in vitro* and *in vivo* were shown to express TLR2, TLR3, and TLR4, but other TLRs were weakly expressed or absent. Using a PCR array, the study demonstrated that, after stimulation with respective TLR ligands, signal transduction through the adaptor protein MyD88 activated downstream factors such as nuclear factor κB (NFκB), interferon regulatory factor (IRF) family, and proinflammatory cytokines and chemokines. To demonstrate the functional response of melanoma cells associated with TLRs, the inventors performed cell migration assays with TLR2, TLR3, or TLR4 ligand. TLR2, TLR3, and TLR4 can induce migration of melanoma cells.

Melanoma cells expressing TLR may promote inflammatory responses in the tumor microenvironoment. The inventors discovered functional and inflammatory activity role of TLR in human cutaneous melanomas. These findings suggest that melanoma cells bearing TLRs may be responsive and supportive of inflammatory responses in the tumor microenvironoment.

Accordingly, the invention provides a method of detecting TLR gene expression or protein activity in a melanocyte or melanoma cell. Melanocytes or melanoma cells may be obtained from cell cultures or tissue specimens using any of the methods known in the art. As described in detail below, melanoma cells may also be isolated from tumor biopsy specimens using HMW-MAA mAb, a cell surface antigen, and Dynabeads CELLection Pan Mouse IgG Kit (Invitrogen) for melanoma tissue cell suspension.

TLR genes and proteins are known in the art. More specifically, a TLR gene or protein may be TLR1, 2, 3, 4, 5, 6, 7, 8, 9, or 10. The expression of a gene can be detected and quantified at mRNA or protein level using a number of means well known in the art.

To measure mRNA levels, cells in biological samples (e.g., cultured cells and tissues) can be lysed and the mRNA levels in the lysates or in RNA purified or semi-purified from the lysates determined by any of a variety of methods familiar to those in the art. Such methods include, without limitation, hybridization assays using detectably labeled gene-specific DNA or RNA probes and quantitative or semi-quantitative real-time RT-PCR methodologies using appropriate gene-specific oligonucleotide primers. Alternatively, quantitative or semi-quantitative in situ hybridization assays can be carried out using, for example, unlysed tissues or cell suspensions, and detectably (e.g., fluorescently or enzyme-) labeled DNA or RNA probes. Additional methods for quantifying mRNA levels include RNA protection assay (RPA), cDNA and oligonucleotide microarrays, and colorimetric probe based assays. Reverse-transcription polymerase chain reaction (RT-PCR) and quantitative real-time reverse-transcription polymerase chain reaction (qRT) procedures are described in detail below.

Methods of measuring protein levels in biological samples are also known in the art. Many such methods employ antibodies (e.g., monoclonal or polyclonal antibodies) that bind specifically to target proteins. In such assays, an antibody itself or a secondary antibody that binds to it can be detectably labeled. Alternatively, the antibody can be conjugated with biotin, and detectably labeled avidin (a polypeptide that binds to biotin) can be used to detect the presence of the biotinylated antibody. Combinations of these approaches (including "multi-layer sandwich" assays) familiar to those in the art can be used to enhance the sensitivity of the methodologies. Some of these protein-measuring assays (e.g., ELISA or Western blot) can be applied to lysates of test cells, and others (e.g., immunohistological methods or fluorescence flow cytometry) applied to unlysed tissues or cell suspensions. Methods of measuring the amount of a label depend on the nature of the label and are known in the art. Appropriate labels include, without limitation, radionuclides (e.g., ¹²⁵I, ¹⁸¹I, ³⁵S, ³H, or ³²P), enzymes (e.g., alkaline phosphatase, horseradish peroxidase, luciferase, or β-glactosidase), fluorescent moieties or proteins (e.g., fluorescein, rhodamine, phycoerythrin, GFP, or BFP), or luminescent moieties (e.g., Qdot™ nanoparticles supplied by the Quantum Dot Corporation, Palo Alto, CA). Other applicable assays include quantitative immunoprecipitation or complement fixation assays. Immunostaining procedures are described in detail below.

The activity of a TLR protein may be detected using any of the methods well known in the art. For example, the activity of a TLR protein may be detected by the binding and activation of the TLR protein by its ligand. The ligands of TLRs are known in the art. For example, zymosan is a ligand of TLR2, poly-inosinic acid:poly-cytidylic acid (PIC) and mRNA from human tumor cells and lymphocytes are ligands of TLR3, and lipopolysaccharide (LPS) is a ligand of TLR4. Other TLR ligands include, but are not limited to, fibrinogen, surfactant protein-A, fibronectin extra domain A, heparan sulfate, soluble hyaluronan, and β-defensin 2 as ligands of TLR4; heat shock protein60 (Hsp60), Hsp70, gp96, and high mobility group box 1 protein as ligands of TLR2 and TLR4; and mRNAs released from necrotic cells as ligands of TLR3. Generally, a melanocyte or melanoma cell is contacted with a TLR ligand under conditions that allow the formation of a ligand-TLR complex. The complex is detected using any of the methods known in the art. For example, a fluorescence microscopy procedure is described in detail below. The activation of the TLR protein may be detected by any of the methods known in the art, e.g., those described in detail below.

The activity of a TLR protein may also be detected by the increased expression of the TLR gene, another TLR gene, a TLR adaptor gene, or a TLR effector gene upon the binding and activation of the TLR protein by its ligand or contacting the cell with a supernatant from phytohemagglutinin-L (PHA-L)-treated peripheral blood lymphocytes (PBL) cells.

A "TLR adaptor" refers to a protein that interacts with the TLR and facilitates signal transduction from the TLR. TLR adaptors are known in the art. Exemplary TLR adaptors include MyD88, CD14, TIRAP, TIRP, TOLLIP, and TRIF.

A "TLR effector" refers to a TLR signal transduction molecule. TLR effectors are known in the art. Exemplary TLR effectors include NFkB1, NFkB2, IRF1, and IRF3.

Briefly, a melanocyte or melanoma cell is contacted with a TLR ligand or a supernatant from phytohemagglutinin-L (PHA-L)-treated peripheral blood lymphocytes (PBL) cells. After the stimulation of the cell, the expression of the TLR gene, another TLR gene, a TLR adaptor gene, or a TLR effector gene is determined using any of the methods described above. Compared to a non-stimulated cell, the expression of the TLR gene, the other TLR gene, the TLR adaptor gene, or the TLR effector gene is increased in the stimulated cell. A PCR array procedure is described in detail below.

Furthermore, the activity of a TLR protein may be detected by the increased expression of a signaling gene downstream of TLR upon the binding and activation of the TLR protein by its ligand.

A "signaling gene downstream of TLR" refers to a transduction pathway gene activated through TLR. Signaling genes downstream of TLR are known in the art. The expression of these genes or activities of the proteins encoded by these genes can be blocked by a compound that binds but does not activate a specific TLR. Exemplary signaling genes downstream of TLR include, but are not limited to, proinflammatory cytokine genes such as IL6, TNFα, IL1, IFNα, IFNβ, and G-CSF, chemokine genes such as CCL2 and CXCL10, anti-inflammatory cytokine genes such as IL10, oncogenes such as JUN, and other genes such as COX-2.

Briefly, a melanocyte or melanoma cell is contacted with a TLR ligand. After the stimulation of the cell, the expression of a signaling gene downstream of TLR is determined using any of the methods described above. Compared to a non-stimulated cell, the expression of the signaling gene downstream of TLR is increased in the stimulated cell. A PCR array procedure is described in detail below.

Alternatively, the activity of a TLR protein may be detected by the increased migration of a melanocyte or melanoma cell upon the binding and activation of the TLR protein by its ligand. Generally, a melanocyte or melanoma cell is contacted with a TLR ligand. After the stimulation of the cell, the migration of the cell is determined using any of the methods known in the art. Compared to a non-stimulated cell, the migration of the stimulated cell is increased. A cell migration assay is described in detail below.

The invention also provides a method of modulating TLR gene expression or protein activity in a melanocyte or melanoma cell in vitro or in vivo by contacting the cell with a TLR modulating agent. A melanocyte or melanoma cell may be obtained and TLR gene expression or protein activity may be determined as described above.

A "TLR modulating agent" refers to a compound that increases or decreases the expression of a TLR gene or the activity of a TLR protein in a cell. A TLR modulating agent may modulate the interaction between a TLR protein and a TLR ligand, adaptor, or effector, or a signaling gene downstream of TLR. For example, a TLR modulating agent may increase or decrease the binding of a TLR protein to its ligand, the expression of a TLR adaptor or effector gene, or the expression of a signaling gene downstream of TLR. TLR modulating agents are known in the art. For example, TLR agonists and antagonists are available from InvivoGen, San Diego, CA.

Alternatively, a TLR modulating agent may be identified by screening a library of compounds. Briefly, a melanocyte or melanoma cell is contacted with a test compound. The expression of a TLR gene or the activity of a TLR protein in the cell prior to and after the contacting step are compared. If the expression of the TLR gene or the activity of the TLR protein in the cell changes (increases or decreases) after the contacting step, the test compound is identified as a TLR modulating agent.

The test compounds can be obtained using any of the numerous approaches (e.g., combinatorial library methods) known in the art. See, e.g., U.S. Patent No. 6,462,187. Such libraries include, without limitation, peptide libraries, peptoid libraries (libraries of molecules having the functionalities of peptides, but with a novel, non-peptide backbone that is resistant to enzymatic degradation), spatially addressable parallel solid phase or solution phase libraries, synthetic libraries obtained by deconvolution or affinity chromatography selection, and the "one-bead one-compound" libraries. Compounds in the last three libraries can be peptides, non-peptide oligomers, or small molecules. Examples of methods for synthesizing molecular libraries can be found in the art. Libraries of compounds may be presented in solution, or on beads, chips, bacteria, spores, plasmids, or phages.

The invention further provides a method of inhibiting melanoma cell migration in vitro or in vivo (e.g., metastasis) by contacting the cell with a TLR inhibitor. A TLR inhibitor decreases the expression of a TLR gene or the activity of a TLR protein in a cell, for example, by inhibiting the interaction between a TLR protein and a TLR ligand, adaptor, or effector, or a signaling gene downstream of TLR. TLR inhibitors are known in the art, or may be identified as described above. A melanoma cell may be obtained and cell migration may be determined as described above.

The discovery of TLRs on melanoma cells is useful for treating melanoma. The TLRs may be used as diagnostic markers for targeted treatment of melanoma or used as prognostic markers. Further, inhibitor molecules of specific TLRs may shut inflammatory and suppressive cytokines, thereby allowing host immunity to be more effective.

For example, to use TLRs as diagnostic markers for targeted treatment of melanoma, a subject suffering from melanoma is identified. A melanoma tumor specimen is obtained from the subject, and the expression of a TLR gene or the activity of a TLR protein in melanoma cells is determined using any of the methods described above. If the expression of the TLR gene or the activity of the TLR protein is detected in the melanoma cells, the subject is identified as a candidate for a TLR-targeting therapy.

Similarly, to use TLRs as prognostic markers, a subject suffering from melanoma is identified. A melanoma tumor specimen is obtained from the subject, and the expression of a TLR gene or the activity of a TLR protein in melanoma cells is determined using any of the methods described above. If the expression of the TLR gene or the activity of the TLR protein is detected in the melanoma cells, the subject is likely to suffer from an invasive or metastatic melanoma.

For treatment of melanoma, a subject suffering from melanoma is identified, and an effective amount of a TLR inhibitor is administered to the subject. TLR inhibitors are known in the art, or may be identified as described above. These inhibitors can be used to inhibit melanoma metastasis and tumor progression.

As used herein, a "subject" refers to a human or animal, including all mammals such as primates (particularly higher primates), sheep, dog, rodents (e.g., mouse or rat), guinea pig, goat, pig, cat, rabbit, and cow. In a preferred embodiment, the subject is a human. In another embodiment, the subject is an experimental animal or animal suitable as a disease model. A subject suffering from melanoma may be identified using any test or diagnostic method known in the art.

A "treatment" is defined as administration of a substance to a subject with the purpose to cure, alleviate, relieve, remedy, prevent, or ameliorate a disorder, symptoms of the disorder, a disease state secondary to the disorder, or predisposition toward the disorder.

An "effective amount" is an amount of a compound that is capable of producing a medically desirable result in a treated subject. The medically desirable result may be objective (i.e., measurable by some test or marker) or subjective (i.e., subject gives an indication of or feels an effect).

For treatment of cancer, a compound is preferably delivered directly to tumor cells, e.g., to a tumor or a tumor bed following surgical excision of the tumor, in order to treat any remaining tumor cells. For prevention of cancer invasion and metastases, the compound can be administered to, for example, a subject that has not yet developed detectable invasion and metastases but is found to have detectable expression of a TLR gene or activity of a TLR protein.

In some embodiments, polynucleotides (i.e., antisense nucleic acid molecules, ribozymes, and siRNAs) are administered to a subject. Polynucleotides can be delivered to target cells by, for example, the use of polymeric, biodegradable microparticle or microcapsule devices known in the art. Another way to achieve uptake of the nucleic acid is using liposomes, prepared by standard methods. The polynucleotides can be incorporated alone into these delivery vehicles or co-incorporated with tissue-specific or tumor-specific antibodies. Alternatively, one can prepare a molecular conjugate composed of a polynucleotide attached to poly-L-lysine by electrostatic or covalent forces. Poly-L-lysine binds to a ligand that can bind to a receptor on target cells. "Naked DNA" (i.e., without a delivery vehicle) can also be delivered to an intramuscular, intradermal, or subcutaneous site. A preferred dosage for administration of polynucleotide is from approximately 10⁶ to 10¹² copies of the polynucleotide molecule.

TLR inhibitors can be incorporated into pharmaceutical compositions. Such compositions typically include the compounds and pharmaceutically acceptable carriers. "Pharmaceutically acceptable carriers" include solvents, dispersion media, coatings, antibacterial and antifungal agents, isotonic and absorption delaying agents, and the like, compatible with pharmaceutical administration.

A pharmaceutical composition is formulated to be compatible with its intended route of administration. See, e.g., U.S. Patent No. 6,756,196. Examples of routes of administration include parenteral, e.g., intravenous, intradermal, subcutaneous, oral (e.g., inhalation), transdermal (topical), transmucosal, and rectal administration. Solutions or suspensions used for parenteral, intradermal, or subcutaneous application can include the following components: a sterile diluent such as water for injection, saline solution, fixed oils, polyethylene glycols, glycerine, propylene glycol or other synthetic solvents; antibacterial agents such as benzyl alcohol or methyl parabens; antioxidants such as ascorbic acid or sodium bisulfite; chelating agents such as ethylenediaminetetraacetic acid; buffers such as acetates, citrates or phosphates; and agents for the adjustment of tonicity such as sodium chloride or dextrose. pH can be adjusted with acids or bases, such as hydrochloric acid or sodium hydroxide. The parenteral preparation can be enclosed in ampoules, disposable syringes, or multiple dose vials made of glass or plastic.

It is advantageous to formulate oral or parenteral compositions in dosage unit form for ease of administration and uniformity of dosage. "Dosage unit form," as used herein, refers to physically discrete units suited as unitary dosages for the subject to be treated, each unit containing a predetermined quantity of an active compound calculated to produce the desired therapeutic effect in association with the required pharmaceutical carrier.

The dosage required for treating a subject depends on the choice of the route of administration, the nature of the formulation, the nature of the subject's illness, the subject's size, weight, surface area, age, and sex, other drugs being administered, and the judgment of the attending physician. Suitable dosages are in the range of 0.01-100.0 mg/kg. Wide variations in the needed dosage are to be expected in view of the variety of compounds available and the different efficiencies of various routes of administration. For example, oral administration would be expected to require higher dosages than administration by intravenous injection. Variations in these dosage levels can be adjusted using standard empirical routines for optimization as is well understood in the art. Encapsulation of the compound in a suitable delivery vehicle (e.g., polymeric microparticles or implantable devices) may increase the efficiency of delivery, particularly for oral delivery.

In summary, specific TLRs, such as TLR2, 3, and 4 are found on melanoma cells at high levels. When activated by their respective specific ligands, proinflammatory genes, chemokines, and other genes are turned on. The activation of these TLRs with Specific ligands can induce tumor cell migration. Activation of TLR induces suppressor cytokine; IL-10. These functions are important in augmenting tumor growth and metastasis. Other TLRs such as TLR5, 6, 7, 8, and 9 are activated on melanoma cells in lower and less frequent levels. The blockade of these receptors' functions on melanoma cells in patients can lead to improvement in host responses, inhibition of metastasis and tumor progression, and a cure of melanoma. This is based on the finding that specific activation of TLR2, 3, and 4 activates several signal transduction genes. The profile of TLR on melanoma cells can improve prognosis and treatment strategy such as targeted therapy. Specific molecules that bind to TLRs but do not activate the TLRs can be used to target human melanoma by blocking tumor growth and activation of the TLRs, e.g., through competition with ligands of the TLRs.

The following example is intended to illustrate, but not to limit, the scope of the invention. While such examples are typical of those that might be used, other procedures known to those skilled in the art may alternatively be utilized. Indeed, those of ordinary skill in the art can readily envision and produce further embodiments, based on the teachings herein, without undue experimentation.

### Example - Expression and Functional Activity of Toll-like Receptors (TLRs) By Human Melanoma Cells

### INTRODUCTION

The inventors hypothesized that melanoma cells express functional TLR and produce proinflammatory cytokines and chemokines through activation of specific TLRs. The present invention focuses on the assessment of TLR expression and functional activity in human melanoma cells. The present invention demonstrates that specific TLRs are expressed and functionally active on melanoma cells. Activation of specific TLRs on melanoma could significantly activate the MyD88 signal transduction pathway, NFκB, and the production of proinflammatory cytokines and chemokines. The present invention indicates that melanoma cells may be responsive and supportive of inflammatory responses in the tumor microenvironment.

### MATERIALS AND METHODS

### Cell Lines and Tissues

Established human metastatic melanoma cell lines used in the present invention were as follows. Melanoma lines: ME-01, ME-02, ME-05, ME-07, ME-08, ME-09, ME-10, ME-15, ME-16, ME-17, ME-18, ME-19, ME-20, ME-21, ME-22, and ME-23. Other established human cell lines were assessed as controls included: breast cancer, MCF-7 and T-47D; colon cancer, SW480; pancreatic cancer, PANC-1; normal cell line, HUVEC (human umbilical vein endothelial cells); human fibroblasts, HFB-O and HFB-S; normal donor peripheral blood lymphocytes (PBL) from normal consenting healthy donors, PBL-A, PBL-B, PBL-1, PBL-2, PBL-3, PBL-4, PBL-5, PBL-6, PBL-A1, PBL-A2, PBL-A3, and PBL-A4; and human-cultured melanocytes, HEnM-MP (Cascade Biologics, Portland, OR). All cell lines except PANC-1 and human-cultured melanocytes were grown in GIBCO RPMI 1640 (Invitrogen, Carlsbad, CA) medium supplemented with 10% heat-inactivated fetal bovine serum (FBS). PANC-1 was grown in DMEM with 10% heat-inactivated FBS. The human-cultured melanocytes were grown in Medium 254 (Cascade Biologics) containing Human Melanocyte Growth Supplement (Cascade Biologics). All cell lines were cultured at 37°C, with a humidified atmosphere containing 5% CO₂ as previously described (12).

### Flow Cytometry

Flow cytometry analyses were performed on BD FACSCalibur System (BD Biosciences, San Jose, CA). 1x10⁶ cells were stained separately with a monoclonal antibody (mAb) to 3 µg of TLR2-PE, TLR3-PE or TLR4-PE (IMGENEX, San Diego, CA). PE-conjugated mouse IgG1 and IgG2a (BD Biosciences) as isotype controls relevant for each Ab were used. Cells were fixed in 4% formaldehyde and incubated for 30 min at 4°C with direct-conjugated mAbs. To block nonspecific binding, phosphate buffered saline (PBS), pH 7.4 (Invitrogen), supplemented with 1% FCS was used for all labeling and washing steps. All flow cytometry data was analyzed using Cell Quest software (Becton Dickinson, Franklin Lakes, NJ).

### Fluorescence Microscopy

Cells were fixed with 4% paraformaldehyde in PBS for 10 min, and then incubated inside a 37°C humidified atmosphere containing 5% CO₂ for 20 min in RPMI 1640 medium supplemented with 10% FBS and 20 µg/ml of fluorescein isothiocyanate (FITC)-LPS (Sigma, St Louis, MO). After washing, cells were visualized using an Olympus IX70 fluorescence microscope. The images were recorded using an Olympus C-3030 Zoom Camedia Digital Camera.

### RNA Isolation, Primers and Probes

Total RNA from cells was extracted using Tri-Reagent (Molecular Research Center Inc., Cincinnati, OH) as previously described (13, 14). RNA extraction procedures were performed in a designated sterile laminar flow hood with RNase-free labware. Isolated RNA was quantified and assessed for quality and purity by UV spectrophotometry and RIBOGreen detection assay (Molecular Probes, Eugene, OR) as previously described (15). RNA extraction, RT-PCR assay set up, and post-RT-PCR product analysis were carried out in separate rooms designated for each respective procedure to prevent cross-contamination (16).

Primer and probe sequences (Table 1) were designed for reverse-transcription polymerase chain reaction (RT-PCR) and qRT, as previously described (17). Specific primers were designed to amplify at least one exon-intron-exon region. Initially, all RT-PCR products were assessed by gel electrophoresis to confirm the amplicon size and visually assess the product specificity.

**Table 1A. Primers Used for RT-PCR and qRT Analyses**

| **mRNA** | **Forward Primer** | **Reverse Primer** |
|---|---|---|
| GAPDH | 5'-GGGTGTGAACCATGAGAAGT-3' | 5'-GACTGTGGTCATGAGTCCT-3' |
| TLR1 | 5'-AGTTGTCAGCGATGTGTTCGG-3' | 5'-GATCAAGTACCTTGATCOTGGG-3' |
| TLR2 | 5'-TCAACTGGTAGTTGTGGGTTG-8' | 5'-CAAGAGAGAGAAGCCTGATTG-3' |
| TLR3 | 5'-GACCCATTATGCAAAAGATTCA-3' | 6'-GCAAACAGAGTGCATGGTTC-3' |
| TLR4 | 5'-GGATTTATCCAGGTGTGA-3' | 6'-TCCCAGGGCTAAACTOT-3' |
| TLR5 | 5'-ACAACTTAGCACGGCTCTGGA-3' | 5'-GAAACCCCAGAGAACGAGTCAG-3' |
| TLR6 | 5'-ATCCTCATGCCTTCAGGAAA-3' | 5'-ACCAGAAGAGACTGGGCTGT-3' |
| TLR7 | 5'-GATAACAATGTCACAGCCGTCC-3' | 5'-GTTCCTGGAGTTTGTTGATGTTC-3' |
| TLR8 | 5'-GTGTCACCCAAACTGCCAAGCTCC-3' | 6'-GATCCAGCACCTTCAGATGAGGC-3' |
| TLR9 | 5'-TACCAACATCCTGATGCTAGACTC-3' | 5'-TAGGACAACAGCAGATACTCCAGG-3' |
| TLR10 | 5'-TATGACAGCAGAGGGTGATGC-3' | 5'-TGCGGGAACCTTTCTTAGAGA-3' |
| MART-1 | 5'-AAAACTGTGAACCTGTGGT-3' | 5'-TTCAGCAAAAGTGTGAGAGA-3' |
| CD45 | 5'-ATTCACTGCAGGGATGGATCT-3' | 5'-ATTGCTCGAATGTGGAAACC-3' |
| CD3-_{Y} Cytokeratin | 5'-TCGAGAGCTTCAGACAAGCA-3' | 5'-TTCCTCCTCAACTGGTTTCC-3' |
| 20 | 5'-ACTTACCGCCGCCTTCT-3' | 5'-CGACCTTGCCATCCACT-3' |
| CD34 | 5'-GCTGGGGATCCTAGATTTCA-3' | 5'-CCAGCCTTTCTCCTGTGG-3' |
| MyD88 | 5'-CCTCTGTAGGCCGACTGCT-3' | 5'-TTGGCAATCCTCOTCAATG-3' |

**Table 1B. Probes Used for RT-PCR and qRT Analyses**

| mRNA | Probe |
|---|---|
| GAPDH | 5'-FAM-CAGCAATGCCTCCTGCACCACCAA-BHQ-1-3' |
| TLR2 | 6'-FAM-TTCTTCCTTGGAGAGGCTGATGATG-BHQ-1-3' |
| TLR3 | 5'-FAM-CTGGAATCTCCTCAAGGAAAACCAA-BHQ-1-3' |
| TLR4 | 5'-FAM-TCCTGTCAATATTAAGGTAGAGAGG-BHQ-1-3' |
| MyD88 | 5'-FAM-TGCTTTACCAAGCTGGGCCGC-BHQ-1-3' |

### RT-PCR and PCR Array

Reverse-transcriptase reactions were performed on 1.0 µg of extracted total RNA from cells using Moloney murine leukemia virus reverse-transcriptase (Promega, Madison, WI, USA) with oligo-dT primers, as previously described (17). The RT-PCR assay conditions for TLR1, 2, 3, 4, 5, 6, 7, 8, 9, and 10 were as follows: one cycle of denaturing at 95°C for 5 min, followed by 30 cycles of 95°C for 1 min, annealing for 1 min at 60°C for TLR1, TLR3, TLR5, TLR7, TLR8, TLR9, and TLR10; 63°C for TLR2 and TLR6; 54°C for TLR4; 55°C for GAPDH; and 72°C for 1 min before a final extension at 72°C for 10 min. The RT-PCR conditions of melanoma cells in *vivo* captured by beads of high molecular weight melanoma-associated antigen (HMW-MAA) mAb were as follows: 1 cycle of denaturing at 95°C for 5 min, followed by 35 cycles of 95°C for 1 min, annealing for 1 min at 56°C for Cytokeratin 20 (CK20); 59°C for Melanoma Antigen Recognized by T-cells 1 (MART-1); 62°C for CD3_{Y}; 63°C for CD45, CD34, and 55°C for GAPDH; and 72°C for 1, min before a final extension at 72°C for 10 min. The specificity of PCR conditions and annealing temperatures for each marker were optimized using an Omni thermocycler (Hybaid, Middlesex, UK). The RT-PCR cDNA products were run on a 2% agarose gel. The qRT assay was performed on the iCycler iQ RealTime PCR Detection System (Bio-Rad Laboratories, Hercules, CA) using 250 ng of total RNA for each reaction, as previously described (18). The PCR reaction mixture consisted of 0.4 µM of each primer, 0.3 µM *Taq*Man probe, 1 unit of Ampli*Taq* Gold polymerase (Applied Biosystems, Foster City, CA), 200 µM each of deoxynucleotide triphosphate, 4.5 mM MgCl₂, and Ampli*Taq* buffer diluted to a final volume of 25 µL. Samples were amplified with a pre-cycling hold at 95°C for 10 min, followed by 35 cycles of denaturation at 95°C for 1 min, 35 cycles of annealing for 1 min at 55°C for GAPDH, 63°C for TLR2, 60°C for TLR3, and 54°C for TLR4, and extension at 72°C for 1 min. Each sample was assayed in triplicate with positive and reagent controls. Absolute copy numbers were determined by a standard curve with serial dilutions (10⁶-10¹ copies) of DNA containing TLR2, TLR3, and TLR4 cDNA templates. TLR2, TLR3, and TLR4 expression was given as a ratio of TLR2, TLR3, or TLR4/GAPDH mRNA units.

The relative mRNA expression of TLR, adaptors and effectors of TLR, members of the major downstream signaling genes, were analyzed with GEArray Q Series Human Toll-Like Receptor Signaling Pathway Gene Array (PCR array, SuperArray Inc., Bethesda, MD) according to the manufacturer's protocol. PCR Array was performed using cDNA from two melanoma cell lines (ME2 and ME5) non-stimulated and stimulated by TLR2, TLR3, or TLR4 ligand. Cell wall from *Saccharomyces cerevisiae* (zymosan) (InVivoGen, San Diego, CA), poly-inosinic acid:poly-cytidylic acid (polyI:C; PIC; synthetic dsRNA) (BD Biosciences), and lipopolysaccharide (LPS) from *Escherichia coli* serotype 0111:4B (Sigma-Aldrich, St. Louis, MO), were used as ligands for TLR2, TLR3, and TLR4, respectively, to activate melanoma cells. 5x10⁵ cells were incubated in RPMI 1640 medium supplemented with 2.5% heat-inactivated FBS (2.5% FBS RPMI) as a negative control, or 2.5% FBS RPMI with one of the following ligands: LPS (1 µg/ml) for 4 hr, PIC (100 µg/ml) for 24 hr, or zymosan (10 µg/ml) for 18 hr at 37°C in a humidified atmosphere with 5% CO₂. After incubation, cells were washed with PBS, and total RNA extraction and reverse-transcriptase reaction were performed as described above. Briefly, the PCR array was performed on the iCycler iQ RealTime PCR Detection System using 10 ng of total RNA and PCR master mix for SYBR Green detection for each reaction (2, 19). Samples were amplified with a pre-cycling hold at 95°C for 10 min, followed by 40 cycles of denaturation at 95°C for 15 sec, 40 cycles of annealing for 1 min at 60°C. Melanoma cells stimulated by LPS, PIC, and zymosan were compared to non-stimulated cells as controls.

### Isolation of Melanoma Cells from Tumor Biopsy Specimens

To investigate melanoma cell TLR expression *in vivo,* the inventors isolated melanoma cells from tumor biopsy specimens using HMW-MAA mAb, a cell surface antigen, and Dynabeads CELLection Pan Mouse IgG Kit (Invitrogen) for melanoma tissue cell suspension (20). Briefly, after surgery, melanoma tumor specimens were collected and immediately minced under sterile conditions at 4°C. The cell suspension was agitated in RPMI 1640 and filtrated through sterile gauze followed by sterile stainless mesh. After washing by centrifugation, the cells were program-frozen at 1°C per minute in a fluid containing RPMI 1640 with 20% gamma globulin-depleted human serum, penicillin, streptomycin, fungizone, and 10% dimethyl sulfoxide, and stored in a liquid nitrogen freezer until use. On the day of experimentation, the cells were thawed rapidly at 37°C. To pre-coat Dynabeads with antibodies, 1x10⁷ washed Dynabeads were incubated with 2 µg HMW-MAA mAb for 30 min at 4°C. The cells were washed with PBS and incubated in the pre-coated Dynabeads for 20 min at 4°C. Dynabeads-bound melanoma cells were then isolated using a magnet. To separate the Dynabeads from the melanoma cells, the cells were incubated in releasing buffer (DNase I, Invitrogen) for 15 min at room temperature, followed by removal of the Dynabeads using a magnet as described by the manufacturer. Isolated melanoma cells were confirmed by light microscopy by staining. Total RNA was extracted from these isolated melanoma cells as described above.

### Cell Migration

To study cell migration, 6.5-mm Transwells with an 8.0-µm pore polycarbonate membranes insert (Corning Incorporated, Corning, NY) were used as described previously (12). Initially, melanoma cells were harvested from culture dishes using trypsin-EDTA and washed twice with PBS. 5x10⁴ cells were resuspended in 2.5% FBS RPMI and seeded into the insert of the Transwell chamber. The lower chamber was filled with either 2.5% FBS RPMI containing one of the following ligands: LPS (10 µg/ml); PIC (1,000 µg/ml), or zymosan (100 µg/ml), or plain 2.5% FBS RPMI as a negative control. The cells were incubated at 37°C for 24 hr in a humidified atmosphere with 5% CO₂ Post-incubation, the transwell chambers were removed and cells that had migrated to the lower chamber were incubated for an additional 6 hr at 37°C. These cells were then fixed in 80% ethanol, washed with PBS, and stained with H&E. The number of cells migrated was counted in ten different fields at 200x magnification using a light microscope by two reviewers, as described previously (12).

### Treatment of Melanoma Cells with PBL Supernatant

**Human subjects gave informed consent as approved by Joint** Institutional Review Board of Saint John's Health Center and John Wayne Cancer Institute prior to the initiation of the study. 5x10⁵ peripheral blood lymphocytes (PBLs) from four normal donor volunteers were cultured in RPMI 1640 supplemented with 5% Human AB Serum (5% HABS RPMI) (Mediatech, Inc, Herndon, VA) with, and without, 20 µg/ml Leucoagglutinin (PHA-L, Sigma-Aldrich) for 72 hr. The supernatants of the cultured PBLs were then collected. 6x10⁵ melanoma cells were incubated with 2.5 ml of 10% FBS RPMI, in addition to either 2.5 ml of the supernatant containing PHA-L (PHA-L stimulated PBL supernatant), 2.5 ml of the supernatant lacking PHA-L (non-stimulated PBL supernatant), or 2.5 ml of plain 5% HABS RPMI (control culture medium) for a period of 72 hr. After incubation, cells were washed with PBS and total RNA was extracted as described above.

### Statistical Analysis

The student's t-test was used to analyze the number of cells migrated in response to TLRs ligands when compared to those cultured with control medium. Analysis of variance (ANOVA) was used to analyze TLR2, TLR3, TLR4, and MyD88 expression in melanoma cells cultured with PHA-L stimulated PBL supernatant compared to non-stimulated PBL supernatant. Analysis was performed using SAS statistical software (SAS Institute, Cary, NC), and all tests were two-sided with a significance level of P<0.05.

### RESULTS

### TLR Expression by Melanoma Lines

The inventors first screened TLR expression in human melanoma cells by RT-PCR and gel electrophoresis. Seven metastatic melanoma lines (ME-02, ME-05, ME-09, ME-15, ME-16, ME-17, and ME-19) were initially assessed for the expression of TLR1 to 10. The TLR expression profile of melanoma lines is shown in Figure 1. All melanoma lines expressed more than two types of TLR. TLR2 was not expressed in ME2 and TLR4 was not expressed in ME-15. However TLR2, TLR3, and TLR4 were strongly expressed in all other melanoma lines. TLR6 was expressed in 2 of 7 melanoma lines. Because TLR1, TLR5, TLR7, TLR8, TLR9, and TLR10 were either absent or very weakly expressed in melanoma lines, the inventors focused their efforts on TLR2, TLR3, and TLR4 throughout the studies.

Next, the inventors quantified the expression of TLR2, TLR3, and TLR4 using qRT in 16 human melanoma cell lines and human melanocytes. PBLs were used as positive controls. PANC1 (TLR2-, TLR3-, TLR4-) and MCF7 (TLR3-, TLR4-) were used as negative controls. All qRT assays were normalized by GAPDH mRNA levels, as previously described (18), to uniformly assess results. The results of TLR profile expression by qRT are shown in Figure 2. TLR2 was expressed in 9 of 14 melanoma lines. TLR3 and TLR4 were both expressed in all but one (13 of 14) melanoma line. Interestingly, TLR2, TLR3, and TLR4 were expressed in cultured normal human melanocytes as well. mRNA expression level of TLR2, TLR3, and TLR4 in normal melanocytes was equivalent to that in melanomas. This indicated that expression of TLR on melanoma cells was not tumor-related, but lineage-related.

### TLR on Melanoma Cells

To verify that TLRs are present on the cell surface in melanoma lines, the inventors performed FACS analysis. Melanoma lines ME1 (with low mRNA expression of TLR2, TLR3, and TLR4) and ME2 (with high mRNA expression of TLR2, TLR3, and TLR4) were used for FACS analysis to compare mRNA expression to receptor presence. The analysis showed that TLR2, TLR3, and TLR4 are found on the cell surface of melanoma lines (Figure 3). The inventors also determined that the expression of TLR2, TLR3, and TLR4 mRNA correlated to FACS analysis of protein expression.

To demonstrate direct ligand binding to TLR, the inventors examined LPS, a ligand of TLR4. The inventors employed FITC-LPS to assess binding to TLR4 on the cell surface of melanoma lines. PIC-FITC and zymosan-FITC were not available for assessment. The cell lines were stained with FITC-LPS, which demonstrated direct binding of LPS to melanoma cells (Figure 4). PBLs were stained with more intensity with FITC-LPS than melanoma cells, as expected. The FITC-LPS analysis confirmed TLR4 receptor staining on the melanoma cell surface.

### Expression of TLR on Melanoma Cells from In Vivo

The inventors investigated TLR on melanoma cells isolated from melanoma tumor specimens *in vivo.* Assessment of melanoma tumor specimens for TLR expression is difficult, since melanomas have varying levels of tumor infiltrating hemopoietic cells and endothelial cells that would express TLR. To address this problem and to determine if *in vivo* tumor cells express TLR, the inventors isolated melanoma cells from nine single-cell-suspensions derived from melanoma tissues using HMW-MAA mAb-coated beads, as described in Materials and Methods. MART-1 (melanoma marker), CD45 (leukocyte-common antigen), CD3-_{Y} (T-cell antigen receptor complex), CK20 (endothelial antigen), and CD34 (hematopoietic progenitor cell antigen) markers were assessed by RT-PCR to verify that only melanoma cells were isolated. RT-PCR using MART-1, CD45, CD3-_{Y}, CK20, and CD34 primers confirmed that only melanoma cells were isolated (Figure 5). The inventors then performed qRT for TLR2, TLR3, and TLR4 mRNA expression on the purified melanoma cells isolated from in *vivo.* The TLR2, TLR3, and TLR4 mRNA expression in purified melanoma cells as assessed by qRT corroborated the analysis of TLR2, TLR3, and TLR4 mRNA expression in melanoma lines (Figure 6). TLR2, TLR3, and TLR4 expression were present in all 9 samples of purified melanoma cells, except for TLR2 expression, which was absent in one patient specimen. The average mRNA copy numbers of TLR2, TLR3, and TLR4 expressed in purified melanoma cells were at the same level as those in melanoma cell lines. These findings indicate that the expression patterns of TLR2, TLR3, and TLR4 in melanoma cells *in vivo* are consistent with their expression in melanoma lines.

### PCR Array Analysis of TLR Activated Genes

Studies have indicated that innate immune cells produce proinflammatory cytokines in the microenvironment of chronic inflammation upon activation of specific TLRs. Therefore, the inventors hypothesized that melanoma cells may produce proinflammatory cytokines by activation of TLRs in the microenvironment of melanomas. To demonstrate the activation of genes which correlate with inflammation during TLR-ligand stimulation on melanoma cells, the inventors performed a PCR array analysis including 84 genes related to TLR-mediated signal transduction and inflammatory cytokines using cDNA from two melanoma cell lines (ME-2 and ME-5), which were both stimulated and non-treated with LPS, PIC, and zymosan, respectively.

The expression profiles of TLRs and TLR adaptor and effector molecules in the PCR array analysis are shown in **Table 2.** The activation of mRNA expression in melanoma cells incxeased > 2-fold when stimulated with LPS, PIC, or zymosan, compared to those of non-stimulated cells. As evidenced by the expression profiles of TLRs and their adaptor molecules, the expression of TLR3, TLR4, TLR9, CD14 (a co-receptor for LPS), and MyD88 (a TLR proximal adaptor that acts to transduce signals) were elevated after stimulation with LPS, PIC, and zymosan. Notably, the expression of MyD88 increased > 8-fold in treated cells over untreated cells upon LPS, PIC, and zymosan stimulation.

To verify these observations using an independent, more quantitative assay, the inventors performed qRT to assess MyD88 expression. MyD88 expression increased 5.7-, 6.3-, and 4.4-fold in ME2, and 9.1.-, 5.0-, and 10.5-fold in ME5 after stimulation with specific ligands for TLR2, TLR3, and TLR4, respectively. These results confirmed the expression profile analysis of the PCR array (**Figure 7**). After stimulation with TLR ligands, TLRs signal through adaptor protein of MyD88 to activate downstream signal transduction pathway, such as NFκB and IRF family (21). NFκB1 was elevated > 8-fold, 4-fold, and 2-fold after stimulation with LPS, PIC, and zymosan, respectively. NFxB2 was also elevated > 2-fold after stimulation with PIC and zymosan, respectively. IRF1 increased > 4-, 2-, and 4-fold in expression after stimulation with LPS, PIC, and zymosan, respectively. IRF3 expression was elevated > 8-and 2-fold after stimulation with LPS and zymosan, respectively.

**Table 2. The Expression Profiles of TLRs, Their Adaptor Genes, and Effector Genes in a PCR Array**

| **Ligandst†** | **Hold** | **TLR and Adaptor of TLR** | **Effector of TLR** |
|---|---|---|---|
| LPS | >8X‡ | TLR,9, MyD88 | NFκB1, IRF3 |
| | >4 X | TLR3, TLR4 | IRF1 |
| | >2 X | CD14 | |
| PIC | >8X | MyD88 | |
| | >4 X | TLR3, TLR4, TLR9 | NFκB1 |
| | >2 X | CD14 | NFκ82, IRF1 |
| Zymosan | >8 X | MyD88 | |
| | >4 X | TLR3, TLR4, TLR9 | IRF1 |
| | >2 X | CD14 | NFκB1, NFκB2, IRF3 |

| | | | |
|---|---|---|---|
| †Ligands of TLRs which stimulated melanoma cells. ‡Hold is calculated expression in stimulated cells / non-stimulated. Hold is average of the expression in ME2 and ME5. | | | |

Analysis of the PCR arrays showed that TLR major downstream signaling molecules were significantly elevated upon stimulation with LPS, PIC, or zymosan compared to those of nonstimulated cells. Proinflammatory-related molecules were assessed before and after stimulation with respective TLR ligands. The proteins were categorized into proinflammatory cytokines, chemokines, anti-inflammatory cytokines, and others (**Table 3**). The proinflammatoxy cytokines, tumor necrosis factor (TNF) α, IL1, IL6, interferon-(IFN) α, IFNβ, IFNγ, and granulocyte colony-stimulating factor (G-CSF), were elevated > 2-fold after stimulation with LPS. Only TNFα expression was elevated > 2-fold with PIC stimulation. G-CSF was elevated > 8-fold, and IL1, TNFα, and IFNβ were elevated > 2-fold after stimulation with zymosan. The expressions of chemokine ligand 2 (CCL2) and chemokine ligand 8 (CXCL10), both of which are considered proinflammatory chemokines, increased > 8 times after stimulation with LPS, PIC, and zymosan. Also, anti-inflammatory cytokine IL10 expression increased > 8 times after stimulation with LPS, PIC, and zymosan. Other molecules, such as cyclooxygenase 2 (COX-2) increased > 4-fold in expression after stimulation with LPS, PIC, and zymosan.

**Table 3: The Expression Profiles of TLR, Downstream Signaling Genes in a PCR Array**

| **Ligands** | | **Proinflammatory Cytokines** | **Chemokines** | **Anti-inflammatory Cytokines** | **Others** |
|---|---|---|---|---|---|
| LPS | >8X | | CCL2, CXCL10 | IL10 | |
| | >4X | IL6 | | | COX-2 |
| | >2X | TNFα, IL1, IFNα, IFNβ, G-CSF | | | |
| PIC | >8 X >8X | | CCL2, CXCL10 | IL10 | |
| | >4 X | | | | COX-2 |
| | >2 X | TNFα | | | |
| Zymosan | >8X | G-CSF | CCL2, CXCL10 | IL10 | |
| | >4X | | | | COX-2 |
| | >2 X | IL1, TNFa, IFNβ | | | |

These results strongly indicate that TLR2, TLR3, or TLR4 stimulated by respective ligands on melanoma cells are responsible for activating inflammatory-related proteins. After stimulation with TLR ligands, TLRs and MyD88 were activated in melanoma cells, and downstream signal transduction pathway, such as NFκB and IRF family were activated by signal transduction from MyD88, leading to the production of inflammatory-related cytokines and chemokines.

### Cell Migration

TLR4 signaling controls the migratory response of polymorphonuclear leukocytes (PMNs) by regulating the sensitivity of a cell surface chemokine receptor (22). The inventors hypothesized that activation of TLRs signaling in melanoma cells can promote migration and facilitate metastasis. To demonstrate the functional response of melanoma cells associated with the interaction of TLR2, TLR3, and TLR4 to their respective ligands, the inventors performed cell migration assays. Melanoma cell lines ME-02, ME-05, and ME-07 were assessed for TLR2/zymosan, TLR3/PIC, and TLR4/LPS : ME-02, ME-05, and ME-07 (TLR2⁺, TLR3⁺, and TLR4⁺). Melanoma cell lines ME-02, ME-05, and ME-07 had significantly greater migration in response to LPS when compared to those cultured with the same medium in the absence of LPS (P=0.001, P=0.0004, and P=0.004, respectively; **Figure 8A**). A similar result was observed when comparing cell lines cultured with PIC and those that were cultured in the absence of PIC (P=0.0008, P=0.0003, and P=0.006, respectively; **Figure 8B**). When assessing zymosan, only ME-05 demonstrated a significant increase in the number of migratory cells (P=0.003). These results indicate that activation of TLR2, TLR3, and TLR4 with respective ligands can induce cell migration of melanoma cells. This suggested that activation of these TLRs may promote invasion and metastasis.

### Melanoma TLR Activation by Activated PBL Supernatant

The inventors hypothesized that activated hemopoietic cells in the microenvironment may promote TLR activation of melanomas and promote expression of inflammatory genes. To address this, an *in vitro* model system of stimulating melanoma cells with activated PBL supernatant was developed. The inventors cultured melanoma cells bearing specific TLRs with supernatant from cultured PBLs stimulated by PHA-L for 72 hrs and assessed TLR2, TLR3, TLR4, and MyD88 expression by melanoma cells using qRT. Four melanoma cell lines (ME1, ME2, ME5, and ME7) were cultured with supernatant from PHA-L stimulated PBLs of four healthy donors. The expression of TLR2 increased 3.7-, 6.6-, 3.1-, and 3.7-fold in ME1, ME2, ME5, and ME7, respectively, after PHA-L stimulated PBL supernatant stimulation compared to non-stimulated PBL supernatant (P=0.018, P=0.0002, P<0.0001, and P<0.0001, respectively, **Figure 9A****).** The expression of TLR3 increased 8,5-, 9.9-, 4.4-, and 6.8-fold for ME1, ME2, ME5, and ME7, respectively (P=0.0002, P<0.0001, P=0.0016, and P<0.0001, respectively, **Figure 9B**). The expression of TLR4 increased 3.9-, 4.0-, 6.2-, and 5.4-fold in ME1, ME2, ME5, and ME7, respectively (P=0.014, P=0.038, P=0.1321, and P<0.0001, respectively, **Figure 9C**). This supported the role of stimulation of hemopoietic cells in activating TLRs on melanoma cells. In addition, the expression of MyD88 which is an adaptor molecule of TLR increased 2.2-, 2.3-, 2.6-, and 3.8-fold in ME1, ME2, ME5, and ME7, respectively, in melanoma cells cultured with PHA-L stimulated PBL supernatant compared to non-stimulated PBL supernatant (P=0.0081, P=0.0045, P=0.0003, and P<0.0001, respectively, **Figure 9D**). There was no difference in the expression of TLR2, TLR3, TLR4, and MyD88 between non-stimulated PBL supernatant and control culture medium. These findings support that TLRs expression on melanoma cells can be enhanced and functionally activated by stimulated hemopoietic cells.

The inventors also discovered that activation of a specific TLR with specific ligand could activate other TLRs by a cooperative mechanism.

Furthermore, activation of TLR 2, 3, and 4 also activated oncogenes such as JUN significantly. Specific ligands such as mRNA from human tumor cells and lymphocytes could activate TLR3 and respective downstream pathways.

### DISCUSSION

Recently, studies have examined the relation between TLR expression of immune cells involved in innate immune responses against cancer (23). However, they have not focused on TLR expression on tumor cells and activation of proinflammatory responses by tumor cells. The inventors' hypothesis was that melanoma cells express and have functional TLR that may play a role in proinflammatory responses. In the present invention, it was demonstrated that TLR2, TLR3, and TLR4 are strongly expressed and functional on human melanoma cells for both cultured *in vitro* cells and *in vivo* isolated cells. Other TLR family members were present but in low frequency and expression level. Interestingly, the inventors found TLR2, TLR3, and TLR4 expressed in normal human melanocytes which suggests that activation of TLR on melanoma cells is lineage-related. The skin is the first barrier against exogenous pathogens and ultraviolet light. Normal langerhans cells and keratinocytes play a part in the antimicrobial defense barrier of human skin through TLRs (24, 25). In addition, studies have indicated that TLRs are expressed in astrocytes and microglia and can induce proinflammatory cytokines (26, 27). TLRs in astrocytes and microglia, endogeneous cells of CNS derived from ectodermal tissue of developing embryos, show similar functions as in melanocytes. Melanocytes may also play a protective role during injury, sunburn, and infection of the skin.

Chronic inflammation is a risk factor for the development of cancer as well as potentiating its progression. Infiltration of tumors with T-cells can be beneficial for cancer patients (28); however extensive analyses of human tumor samples have revealed that the abundance of innate immune cells can also correlate with angiogenesis and poor prognosis (11). Innate immune cells, e.g., granulocytes (neutrophils, basophils, and eosinophils), dendritic cells (DCs), macrophages, natural killer cells (NK cells), and mast cells, have TLRs. Activation of TLRs triggers a cascade of intracellular events, including innate immune responses through NFκB-dependent and IRF-dependent signaling pathways. This results in increased production of proinflammatory mediators, and thus, further recruitment and activation of leukocytes (29).

Until the present invention, studies have indicated that immune cells produce cytokines and chemokines in the environment of chronic inflammation. However, the inventors hypothesized that melanoma cells may be also involved in the production of cytokines and chemokines through activation of tumor cell TLRs. When inflammation occurs in the skin microenvironment of melanoma tumors or melanocytes, various immune factors may be released that activate TLR expression in response. This may be a natural response of melanocytes in the skin. Normal melanocytes were shown to have TLR expression, suggesting that melanocytes may play a role in defense against skin irritation such as UV exposure and inflammation. As demonstrated in the present invention, cytokine and chemokine molecules were elevated in melanoma cells. High levels of TNFα, IL1, IL6, IFNα, IFNβ, and G-CSF (proinflammatory cytokines), CCL2 and CXCL10 (chemokines), and IL10 (anti-inflammatory cytokine) expression were found to be activated in melanoma cells when treated with TLRs ligands. TNFα, IL6, IL1, IFNs, G-CSF, and CCL2 are all major proinflammatory cytokines. TNFα is one of the key cytokines mediating the inflammatory processes during tumor promotion (30). TNFα controls leukocytic infiltration in tumors through modulation of chemokines and their receptors. It is known that melanoma cells produce TNFα (31). Endogenous TNFα chronically produced in the tumor microenvironment enhances tumor growth and invasion by inducing other cytokines/chemokines involved in cancer progression, such as IL6 and CCL2 (32). IL6 acts on myeloid progenitor cells and B and T lymphocytes (33). IL1 is a primary regulator of inflammation that causes leukocyte accumulation; the induction of IL1 leads to the production of several factors, including TNF-alpha, IL6, and IL1 receptor antagonist (IL1Ra), either through cytokine production or signaling pathways (34). Excessive IFN-α and IFN-β secretion leads to chronic inflammation, since IFN-α and IFN-β inhibit activated T-cell apoptosis (35). G-CSF specifically stimulates the proliferation and differentiation of myeloid precursors, such as macrophages, DCs, and granulocytes (36). CC L2 and CXCL10 are considered proinflammatory chemokines. CCL2 recruits macrophages, which accumulate in the tumor site where they adversely impact both the local inflammation outcome and whole patient prognosis (37). The density of tumor-associated macrophage (TAM) correlates with a poor prognosis (11, 37). CCL2 also induces recruitment of monocytes, lymphocytes, eosinophils, and basophils. CXCL10 is a Th1-associated chemokine, which has the potential to recruit Th1-type T cells, and has been recently known to be responsible for the recruitment of mast cells (38). Anti-inflammatory cytokine IL10, a Th2-type pleiotropic cytokine, has been shown to hinder a number of immune functions. IL10-treated DCs induce alloantigen- or peptide-specific anergy in CD4+ and CD8+ T lymphocytes, which are then able to suppress activation and function of other T cells (39).

COX2 has a role as a major mediator of inflammation; enhanced COX2-induced synthesis of prostaglandins stimulates cancer cell proliferation and increases metastatic potential (40). Except IL10, these cytokines and chemokines, which increased in all of the experiments, are produced by melanoma cells. Hence, they enhance recruitment and activation of additional leukocytes and cause inflammation in the microenvironment around the melanoma cells. Moreover, this inflammation may occur by proinflammatory cytokines and chemokines from not only immune cells, but also melanoma cells, and may aid melanoma progression. Activation of the responses may initially be a response to activate inflammation; however as this response becomes more chronic the tumor cells may take advantage to thwart off immune responses.

The present invention demonstrated after stimulation with respective TLR ligands, signal transduction through the adaptor protein MyD88 activated downstream factors such as NFκB and IRF family. NFκB plays a key role in regulating the immune response, and controls many genes involved in inflammation. In addition, studies have shown that activation of NFκB enhances carcinogenesis of inflammation-associated cancer (41, 42). It is known that inflammatory cells are capable of modulating expression of genes associated with proliferation and survival of cancer cells via NFκB regulation (10). The family of IRF transcription factors is important in the regulation of interferons, and IRF1 and IRF3 are transcriptional activators for the type I IFN genes (43, 44). MyD88, NFκB1, NFκB2, IRF1, and IRF3 were significantly stimulated by activation of TLR2, TLR3, and TLR4 in melanoma cells. NFκB and IRF family may play a significant role in regulating cytokine and chemokine production and recruiting immune cells in the microenvironment of melanomas cells.

The expression of TLR2, TLR3, TLR4, and MyD88 significantly increased in melanoma cells cultured with supernatant from PHA-L activated PBLs compared to unstimulated PBLs and control medium. Recently, a number of endogenous molecules have been reported to be ligands of TLRs, such as fibrinogen, surfactant protein-A, fibronectin extra domain A, heparan sulfate, soluble hyaluronan, and β-defensin 2 as ligands of TLR4 (45); and heat shock protein60 (Hsp60), Hsp70, gp96, and high mobility group box 1 protein as ligands of TLR2 and TLR4 (45). The endogenous ligands of TLRs are capable of inducing innate immune response and producing pro-inflammatory cytokines in immune cells. In addition, Kariko et al. showed that mRNAs released from necrotic cells are endogenous ligands for TLR3, and induce immune activations (46), and studies have shown mRNAs can be released by tumor cells (47). Activated PBL supernatant includes endogenous ligands from cells within the tumor, including melanoma cells and hemopoietic cells likely found in the microenvironment of melanomas. Therefore, the inventors postulate that TLRs on melanoma cells may be potentially activated indirectly through endogenous ligands of cells within the tumor.

TLR4 signaling controls the migratory response of PMNs by regulating the sensitivity of the cell surface chemokine receptors (22). Therefore, the inventors hypothesized and investigated the possibility that migratory response is controlled by TLRs signaling in melanoma cells. Interestingly, as a novel finding, ligands of TLR2, TLR3, and TLR4 affected cell migration in melanoma cells, as well as leukocytes. The inventors considered that melanoma cells stimulated by TLR ligands may acquire metastatic ability. The inventors' findings suggest that melanoma cells can be promoted to migrate during inflammatory responses.

In summary, the present invention has shown that TLR2, TLR3 and TLR4 were highly expressed in human melanoma *in vivo* and *in vitro.* Melanoma cells may be activated by endogenous ligands from cells within the tumor stimulating cytokines and chemokines resulting in chronic inflammation of the microenvironment. In addition, stimulation with ligands of TLR2, TLR3, and TLR4 had a progressive effect on cell migration in melanoma cells. This progressive effect is intensified by activated leukocytes in the microenvironment of melanoma cells. The inventors suggest that this may be a positive feedback mechanism to increase and maintain inflammation in the microenvironment of melanoma cells, the key factor being TLRs on melanoma cells. TLRs are thus potential targets for immunotherapy and molecular therapy in melanoma patients.

### REFERENCES

1. Medzhitov R, Preston-Hurlburt P, Janeway CA, Jr. A human homologue of the Drosophila Toll protein signals activation of adaptive immunity. Nature 1997; 388: 394-397.
2. Takeda K, Kaisho T, Akira S. Toll-like receptors. Annu Rev Immunol 2003; 21: 335-376.
3. Tsuji S, Matsumoto M, Takeuchi O, et al. Maturation of human dendritic cells by cell wall skeleton of Mycobacterium bovis bacillus Calmette-Guerin: involvement of toll-like receptors. Infect Immun 2000; 68: 6883-6890.
4. Uehori J, Matsumoto M, Tsuji S, et al. Simultaneous blocking of human Toll-like receptors 2 and 4 suppresses myeloid dendritic cell activation induced by Mycobacterium bovis bacillus Calmette-Guerin peptidoglycan. Infect Immun 2003; 71: 4238-4249.
5. Huang B, Zhao J, Li H, et al. Toll-like receptors on tumor cells facilitate evasion of immune surveillance. Cancer Res 2005; 65: 5009-5014.
6. Kelly MG, Alvero AB, Chen R, et al. TLR-4 signaling promotes tumor growth and paclitaxel chemoresistance in ovarian cancer. Cancer Res 2006; 66: 3859-3868.
7. Balkwill F, Mantovani A. Inflammation and cancer: back to Virchow? Lancet 2001; 357: 539-545.
8. Coussens LM, Werb Z. Inflammation and cancer. Nature 2002; 420: 860-867.
9. Mantovani A. Cancer: inflammation by remote control. Nature 2005; 435: 752-753.
10. de Visser KE, Coussens LM. The interplay between innate and adaptive immunity regulates cancer development. Cancer Immunol Immunother 2005; 54: 1143-1152.
11. Bingle L, Brown NJ, Lewis CE. The role of tumour-associated macrophages in tumour progression: implications for new anticancer therapies. J Pathol 2002; 196: 254-265.
12. Mori T, Kim J, Yamano T, et al. Epigenetic up-regulation of C-C chemokine receptor 7 and C-X-C chemokine receptor 4 expression in melanoma cells. Cancer Res 2005; 65: 1800-1807.
13. Sarantou T, Chi DD, Garrison DA, et al. Mel anoma-associated antigens as messenger RNA detection markers for melanoma. Cancer Res 1997; 57: 1371-1376.
14. Takeuchi H, Fujimoto A, Tanaka M, Yamano T, Hsueh E, Hoon DS. CCL21 chemokine regulates chemokine receptor CCR7 bearing malignant melanoma cells. Clin Cancer Res 2004; 10: 2351-2358.
15. Kuo CT, Hoon DS, Takeuchi H, et al. Prediction of disease outcome in melanoma patients by molecular analysis of paraffin-embedded sentinel lymph nodes. J Clin Oncol 2003; 21: 3566-3572.
16. Koyanagi K, O'Day SJ, Gonzalez R, et al. Serial monitoring of circulating melanoma cells during neoadjuvant biochemotherapy for stage III melanoma: outcome prediction in a multicenter trial. J Clin Oncol 2005; 23: 8057-8064.
17. Takeuchi H, Morton DL, Kuo C, et al. Prognostic significance of molecular upstaging of paraffin-embedded sentinel lymph nodes in melanoma patients. J Clin Oncol 2004; 22: 2671-2680.
18. Goto Y, Matsuzaki Y, Kurihara S, et al. A new melanoma antigen fatty acid-binding protein 7, involved in proliferation and invasion, is a potential target for immunotherapy and molecular target therapy. Cancer Res 2006; 66: 4443-4449.
19. Jiang Z, Zamanian-Daryoush M, Nie H, Silva AM, Williams BR, Li X. Poly(I-C)-induced Toll-like receptor 3 (TLR3)-mediated activation of NFkappa B and MAP kinase is through an interleukin-1 receptor-associated kinase (IRAK)-independent pathway employing the signaling components TLR3-TRAF6-TAK1-TAB2-PKR. J Biol Chem 2003; 278: 16713-16719.
20. Hamby CV, Liao SK, Kanamaru T, Ferrone S. Immunogenicity of human melanoma-associated antigens defined by murine monoclonal antibodies in allogeneic and xenogeneic hosts. Cancer Res 1987; 47: 5284-5289.
21. Dunne A, O'Neill LA. Adaptor usage and Toll-like receptor signaling specificity. FEBS Lett 2005; 579: 3330-3335.
22. Fan J, Malik AB. Toll-like receptor-4 (TLR4) signaling augments chemokine-induced neutrophil migration by modulating cell surface expression of chemokine receptors. Nat Med 2003; 9: 315-321.
23. Tsuji S, Matsumoto M, Takeuchi O, et aL Maturation of human dendritic cells by cell wall skeleton of Mycobacterium bovis bacillus Calmette-Guerin: involvement of toll-like receptors. Infect Immun 2000; 68: 6883-6890.
24. Flacher V, Bouschbacher M, Verronese E, et al. Human Langerhans Cells Express a Specific TLR Profile and Differentially Respond to Viruses and Gram-Positive Bacteria. J Immunol 2006; 177: 7959-7967.
25. Kollisch G, Kalali BN, Voelcker V, et al. Various members of the Toll-like receptor family contribute to the innate immune response of human epidermal keratinocytes. Immunology 2005; 114: 531-541.
26. Bsibsi M, Ravid R, Gveric D, van Noort JM. Broad expression of Toll-like receptors in the human central nervous system. J Neuropathol Exp Neurol 2002; 61: 1013-1021.
27. Jack CS, Arbour N, Manusow J, et al. TLR signaling tailors innate immune responses in human microglia and astrocytes. J Immunol 2005; 175: 4320-4330.
28. Oshikiri T, Miyamoto M, Shichinohe T, et al. Prognostic value of intratumoral CD8+ T lymphocyte in extrahepatic bile duct carcinoma as essential immune response. J Surg Oncol 2003; 84: 224-228.
29. Akira S, Uematsu S, Takeuchi O. Pathogen recognition and innate immunity. Cell 2006; 124(4): 783-801.
30. Szlosarek P, Charles KA, Balkwill FR. Tumour necrosis factor-alpha as a tumour promoter. Eur J Cancer 2006; 42: 745-750.
31. Colombo MP, Maccalli C, Mattei S, Melani C, Radrizzani M, Parmiani G. Expression of cytokine genes, including IL-6, in human malignant melanoma cell lines. Melanoma Res. 1992; 2(3): 181-189.
32. Balkwill F. Tumor necrosis factor or tumor promoting factor? Cytokine Growth Factor Rev 2002; 13: 135-141.
33. Bronte V, Cingarlini S, Marigo I, De Santo C, Gallina G, Dolcetti L, Ugel S, Peranzoni E, Mandruzzato S, Zanovello P. Leukocyte infiltration in cancer creates an unfavorable environment for antitumor immune responses: a novel target for therapeutic intervention. Immunol Invest. 2006; 35(3-4): 327-57.
34. Dinarello CA. Biologic basis for interleukin-1 in disease. Blood 1996; 87: 2095-2147.
35. Akbar AN, Lord JM, Salmon M. IFN-alpha and IFN-beta: a link between immune memory and chronic inflammation. Immunol Today 2000; 21: 337-342.
36. Sebollela A, Cagliari TC, Limaverde GS, Chapeaurouge A, Sorgine MH, Coelho-Sampaio T, Ramos CH, Ferreira ST. Heparin-binding sites in granulocyte-macrophage colony-stimulating factor. Localization and regulation by histidine ionization. J Biol Chem. 2005; 280(36): 31949-31956.
37. Pollard JW. Tumour-educated macrophages promote tumour progression and metastasis. Nat Rev Cancer 2004; 4: 71-78.
38. Morris GE, Parker LC, Ward JR, Jones EC, Whyte MK, Brightling CE, Bradding P, Dower SK, Sabroe I. Cooperative molecular and cellular networks regulate Toll-like receptor-dependent inflammatory responses. FASEB J. 2006; 20(12): 2153-2155.
39. Steinbrink K, Graulich E, Kubsch S, Knop J, Enk AH. CD4(+) and CD8(+) anergic T cells induced by interleukin-10-treated human dendritic cells display antigen-specific suppressor activity. Blood 2002; 99: 2468-2476.
40. Kuwano T, Nakao S, Yamamoto H, et al. Cyclooxygenase 2 is a key enzyme for inflammatory cytokine-induced angiogenesis. Faseb J 2004; 18: 300-310.
41. Greten FR, Eckmann L, Greten TF, et al. IKKbeta links inflammation and tumorigenesis in a mouse model of colitis-associated cancer. Cell 2004; 118: 285-296.
42. Pikarsky E, Porat RM, Stein I, et al. NF-kappaB functions as a tumour promoter in inflammation-associated cancer. Nature 2004; 431: 461-466.
43. Harada H, Willison K, Sakakibara J, Miyamoto M, Fujita T, Taniguchi T. Absence of the type I IFN system in EC cells: transcriptional activator (IRF-1) and repressor (IRF-2) genes are developmentally regulated. Cell 1990; 63: 303-312.
44. Weaver BK, Kumar KP, Reich NC. Interferon regulatory factor 3 and CREB-binding protein/p300 are subunits of double-stranded RNA-activated transcription factor DRAF1. Mol Cell Biol 1998; 18: 1359-1368.
45. Tsan MF. Toll-like receptors, inflammation and cancer. Semin Cancer Biol 2006; 16: 32-37.
46. Kariko K, Ni H, Capodici J, Lamphier M, Weissman D. mRNA is an endogenous ligand for Toll-like receptor 3. J Biol Chem 2004; 279:12542-12550.
47. Lo YM, Chiu RW. The biology and diagnostic applications of plasma RNA. CNAPS III. Ann N Y Acad Sci. 2004; 1022: 135-139.

All publications cited herein are incorporated by reference in their entirety.

Preferably, a method of detecting Toll-like receptor (TLR) gene expression or protein activity in a cell can comprise providing a melanocyte or melanoma cell; and detecting the expression of a TLR gene or the activity of a TLR protein in the cell.

Preferably, the TLR gene or protein is TLR1, 2, 3, 4, 5, 6, 7, 8, 9, or 10.

Preferably, the melanoma cell is isolated from a melanoma tumor specimen.

Preferably, the expression of the TLR gene is detected at the mRNA or protein level.

Preferably, the expression of the TLR gene is detected by reverse -transcription polymerase chain reaction (RT-PCR), quantitative real-time reverse -transcription polymerase chain reaction (qRT), or immunostaining.

Preferably the activity of the TLR protein is detected by the binding and activation of the TLR protein by its ligand.

Preferably, the ligand is selected from the group consisting of zymosan (a ligand of TLR2), poly-inosinic acid:poly-cytidylic acid (PIC) and mRNA from human tumor cells and lymphocytes (ligands of TLR3), and lipopolysaccharide (LPS) (a ligand of TLR4).

Preferably, the activity of the TLR protein is detected by the increased expression of the TLR gene, another TLR gene, a TLR adaptor gene, or a TLR effector gene upon the binding and activation of the TLR protein by its ligand or contacting the cell with a supernatant from phytohemagglutinin-L (PHA-L)-treated peripheral blood lymphocytes (PBL) ceUs.

Preferably, the TLR adaptor gene is selected from the group consisting of MyD88 and CD 14, and the TLR effector gene is selected from the group consisting of NFkB1, NFkB2, IRF1, and IRF3.

Preferably, the activity of the TLR protein is detected by the increased expression of a signaling gene downstream of TLR upon the binding and activation of the TLR protein by its ligand.

Preferably, the signaling gene downstream of TLR is selected from the group consisting of proinflammatory cytokine genes, chemokine genes, anti-inflammatory cytokine genes, COX-2, and oncogenes.

Preferably, the proinflammatory cytokine genes are selected from the group consisting of IL6, TNFα, IL1, IFNα, IFNβ, and G-CSF, the chemokine genes are selected from the group consisting of CCL2 and CXCLIO, the anti-inflammatory cytokine gene is ILIO, and the oncogene is JUN.

Preferably, the activity of the TLR protein is detected by the increased migration of the cell upon the binding and activation of the TLR protein by its ligand.

Preferably, a method of modulating TLR gene expression or protein activity in a cell can comprise providing a melanocyte or melanoma cell; and contacting the cell with a TLR modulating agent, thereby increasing or decreasing the expression of a TLR gene or the activity of a TLR protein in the cell.

Preferably, the TLR gene or protein is TLR1, 2, 3, 4, 5, 6, 7, 8, 9, or 10.

Preferably, the TLR modulating agent modulates the interaction between the TLR protein and a TLR ligand, adaptor, or effector, or a signaling gene downstream of TLR.

Preferably, a method of inhibiting cell migration can comprise providing a melanoma cell; and contacting the cell with a TLR inhibitor that decreases the expression of a TLR gene or the activity of a TLR protein in the cell, thereby inhibiting the migration of the cell.

Preferably, the TLR gene or protein is TLR1, 2, 3, 4, 5, 6, 7, 8, 9, or 10.

Preferably, the TLR inhibitor inhibits the interaction between the TLR protein and a TLR ligand, adaptor, or effector, or a signaling gene downstream of TLR.

## Claims

1. The use of a TLR modulating agent for treating melanoma cancer, wherein a melanocyte or melanoma cell obtained from a melanoma tumor is contacted with the TLR modulating agent, thereby (i) increasing or decreasing the expression of a TLR gene in the melanocyte or melanoma cell; or (ii) increasing or decreasing the activity of a TLR protein in the melanocyte or melanoma cell.

2. The method of claim 1, wherein the TLR gene or protein is TLR1, TLR2, TLR3, TLR4, TLR5, TLR6, TLR7, TLR8, TLR9, or TLR10.

3. The method of claim 1, wherein the TLR modulating agent modulates the interaction between the TLR protein and a TLR ligand, adaptor, or effector, or a signaling gene downstream of TLR.

4. The method of claim 1, wherein the TLR modulating agent is a TLR inhibitor.

5. The method of claim 4, wherein the TLR inhibitor decreases the expression of a TLR gene or the activity of a TLR protein in the melanocyte or melanoma cell.

6. The method of claim 4, wherein the TLR inhibitor inhibits melanoma cell migration.

7. The method of claim 4, wherein the TLR inhibitor inhibits the interaction between the TLR protein and a TLR ligand, adaptor, or effector, or a signaling gene downstream of TLR.
